# EUROPEAN PATENT SPECIFICATION

(11) **EP 3 188 724 B1**
(45) Date of publication and mention of the grant of the patent: **10.11.2021**
(21) Application number: 15832081.2
(22) Date of filing: 14.08.2015
(51) Int. Cl.: A61K 31/195, A61P 35/00

(54) **LIPOSOME ENCAPSULATED AFFINITY DRUG**
LIPOSOMVERKAPSELTES AFFINITÄTSARZNEIMITTEL
MÉDICAMENT À AFFINITÉ ENCAPSULÉ DANS UN LIPOSOME

(30) Priority: 14.08.2014 US 201462037597 P; 09.03.2015 US 201562130493 P; 13.03.2015 US 201562133265 P
(43) Date of publication of application: 12.07.2017
(73) Proprietor: L.E.A.F Holdings Group LLC, Gulph Mills, PA 19428 (US)
(72) Inventor: NIYIKIZA, Clet, Gulph Mills, PA 19428 (US); VARGHESE, Jose, Somerville, MA 02143 (US)
(74) Representative: Cesa, Roberta
(86) International application number: PCT/US2015/045353
(87) International publication number: WO 2016/025882

(56) References cited:
- WO-A1-2011/150392
- WO-A2-2013/008240
- CA-A1- 2 858 741
- US-A1- 2011 229 529
- US-A1- 2013 122 096
- US-A1- 2013 177 570
- US-A1- 2014 120 157
- ANDREAS WAGNER ET AL: "Liposome Technology for Industrial Purposes", JOURNAL OF DRUG DELIVERY, vol. 2011, 20 October 2010 (2010-10-20), pages 1-9, XP055430225, ISSN: 2090-3014, DOI: 10.1155/2011/591325
- WILLIS, RANDALL C.: 'Goog Things in Small Packages: Nantotech Advances are Producing Mega-Reults in Drug Delivery' MODENR DRUG DISCOVERY July 2005, pages 30 - 35, XP055405429
- None

## Description

### RELATED APPLICATIONS

This Application claims the priority of U.S. provisional Application No. 62/037,597 filed August 14, 2014, U.S. provisional Application No. 62/130,493 filed March 9, 2015 and U.S. provisional Application No. 62/133,265 filed March 13, 2015.

### BACKGROUND

Cancer is a very difficult disease to treat due to diversity of cancer type, mechanisms involved in disease progression and patient variability associated with underlying patient genetic make up. Early efforts to treat cancer have involved the use of cytotoxic agents including antifolates. Antifolates refers to a class of molecules that antagonize (i.e., block) the actions of folic acid (vitamin B9). Folic acid's primary function in the body is to serve as a cofactor to various methyltransferases involved in serine, methionine, thymidine and purine biosynthesis. Consequently antifolates inhibit cell division, DNA/RNA synthesis and repair and protein synthesis.

The rationale for introducing antifolates as anti cancer agents was based on folates being important for survival of all dividing cells because folates are essential ingredients for DNA (nucleic acid) synthesis during cell replications. Folate absorption by any cell, normal or cancerous, is primarily mediated by reduced-folate carriers (RFCs), which is an abundant cross-membrane transporter with low affinity for folates.

Because cancer cells are fast-growing cells and thus have a high demand for DNA precursors in the form of folates, they are susceptible to the effects of antifolates. Fast growing normal cells, such as cells that line the gastrointestinal tract and cells of the bone marrow, divide rapidly as well using folates supplied primarily via RFCs. Normal cells are therefore also susceptible to antifolates because the RFC mediated transport mechanisms which antifolates employ to infiltrate and kill cancer cells also have the potential to result in a collateral effect of killing fast-growing normal cells, thereby causing unwanted antifolate- related toxicities.

Antifolates work by interfering with the action of folates, depriving cancer cells of the DNA precursors they need to proliferate, or grow. Antifolates as a class are used for their antiproliferative effect in the treatment of cancer to inhibit cell growth and division, which causes cancer cells to die. The fast replicating cancer cells requiring increased amount of folates compared to most normal cells led to the clinical development of antifolates as anticancer agents almost 70 years ago. However, though antifolate-based therapy was shown to be effective for cancer treatment, their clinical development has often been derailed due to a compelling clinical dilemma. This dilemma stems from two competing clinical dynamics. On one hand, antifolates are designed to be folate mimic molecules with most of them intended to reach cancer cells by using RFCs as the preferred cross-membrane transport mechanism. On the other hand, fast renewing normal tissues in the body such as, for example, the bone marrow or intestinal track tissue cells are, like cancer cells, also highly folate-dependent and use also RFCs as the primary cross-membrane folate cell supply mechanism. The net result of these two clinical dynamics is that bone marrow and gastrointestinal (GI) tract cells, for example, have typically been a very prevalent site of patients' life-threatening antifolate-related toxicities. Some of these toxicities have included mucositis, diarrhea, anemia, neutropenia, and low white blood counts. The consequence of these antifolate-related intractable side effects in patients has been that antifolates exhibiting highly effective cytotoxic or anti-cancer properties have typically failed during their development or have, to date, limited use in clinical practice because these antifolates also tend to have debilitating side effects in the form of unacceptable toxicities in normal cells.

Antifolates as a class remain a promising treatment modality for cancer despite the associated risk of severe and even life-threatening toxicities for patients. The challenge has been to figure out a way to effectively deliver antifolates in a manner that reduces and/or avoids damage to normal cells. Recently, because of the availability of newer alternative therapies for cancer, antifolates have lost favor in comparison to such therapies in spite of the exceptional effectiveness of antifolates in killing cancer cells. In this context the document US2014120157 is relevant.

### BRIEF SUMMARY

A neutral or anionic immunoliposome with affinity and specificity to folate receptor or receptors containing an aqueous bioactive agent such as anti-cancer (antineoplastic) agent is surprisingly effective against cells presenting folate receptors on their cell surface.

In one embodiment, a liposomal antifolate composition according to claim 1 is provided. The liposomal antifolate composition comprises: a liposome including an interior space; a bioactive antifolate agent disposed within said interior space; a PEG attached to an exterior of the liposome; and a targeting moiety comprising a protein with specific affinity for at least one folate receptor, said targeting moiety attached to at least one of the PEG and the exterior of the liposome. For the liposomal antifolate composition of claim 1, the liposome has a zeta potential that is less than or equal to zero and the liposome is anionic or neutral. The PEG may have a number average molecular weight (Mn) of 200 to 5000 daltons.

In accordance with the invention, a liposomal antifolate composition according to claim 21 is also provided. The example liposomal antifolate composition comprises a medium comprising a liposome including an interior space; an aqueous bioactive antifolate agent disposed within said interior space; a targeting moiety comprising a protein with specific affinity for at least one folate receptor, said targeting moiety disposed at an the exterior of the liposome. The medium in this composition may be an aqueous solution. The aqueous solution may comprise at least one cryoprotectants selected from the group consisting of mannitol; trehalose; sorbitol; and sucrose. The liposomal antifolate composition may further comprise a steric stabilizer attached to the exterior of the liposome, wherein the targeting moiety is attached to at least one of the steric stabilizer and the exterior of the liposome. The steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[N-(2-hydroxypropyl) methacrylamide]; amphiphilic poly-N-vinylpyrrolidones; L-amino-acid-based polymer; and polyvinyl alcohol. The PEG may have a number average molecular weight (Mn) of 200 to 5000 daltons.

In any of the example compositions, liposomes, products, kits and methods, the additional features of the following paragraphs may be incorporated.

The liposomal antifolate composition can further comprise at least one of an immunostimulatory agent and a detectable marker disposed on at least one of the PEG and an exterior of the liposome. The liposomal antifolate composition may have a feature wherein the at least one of an immunostimulatory agent and a detectable marker is covalently bonded to at least one of the PEG and the exterior of the liposome. The immunostimulating agent may be at least one selected from the group consisting of protein immunostimulating agent; nucleic acid immunostimulating agent; chemical immunostimulating agent; hapten; and adjuvant. For example, the immunostimulating agent may be fluorescein isothiocyanate (FITC). As another example, the immunostimulating agent is at least one selected from the group consisting of: fluorescein; DNP; beta glucan; beta-1,3-glucan; and beta-1,6-glucan. The detectable marker may be at least one selected from the group consisting of fluorescein and fluorescein isothiocyanate (FITC). As an example, the immunostimulatory agent and the detectable marker is the same - for example, it may be fluorescein isothiocyanate (FITC).

The liposomal antifolate composition may have a diameter in the range of 30-150 nm, such as, for example, in the range of 40-70 nm. As another feature, the liposome can be an anionic liposome or a neutral liposome. The zeta potential of the liposome is less than or equal to zero; for example in the range of 0 to -150 mV or in the range of -30 to -50 mV.

The liposomal antifolate composition comprises liposomes. The liposomes may be formed of any liposomal components. For example, the liposomal component may comprise at least one of an anionic lipid and a neutral lipid. As another example, the liposomal component is at least one selected from the group consisting of: DSPE; DSPE-PEG-maleimide; HSPC; HSPC-PEG; cholesterol; cholesterol-PEG; and cholesterol-maleimide. As another example, the liposomal components comprise at least one selected from the group consisting of: DSPE; DSPE-PEG-FITC; DSPE-PEG-maleimide; cholesterol; and HSPC.

As discussed, the liposome may enclose an aqueous solution. For example, the liposome can enclose a bioactive antifolate agent and an aqueous pharmaceutically acceptable carrier. The pharmaceutically acceptable carrier may comprise trehalose such as, for example, 5% to 20% weight percent of trehalose. The pharmaceutically acceptable carrier, for example, may compris citrate buffer at a concentration of between 5 to 200 mM and a pH of between 2.8 to 6. Independently of other ingredients, the pharmaceutically acceptable carrier may comprise a total concentration of sodium acetate and calcium acetate of between 50 mM to 500 mM.

The bioactive antifolate agent may be water soluble. As an example, the liposomal antifolate composition may have a liposome and some of the liposome may comprise less than 200,000 molecules of the bioactive antifolate agent. For example, the liposome may comprise between 10,000 to 100,000 molecules of the bioactive antifolate agent.

The bioactive antifolate agent may comprise pemetrexed. In another example embodiment, the bioactive antifolate agent may comprise lometrexol. In another example embodiment, the bioactive antifolate agent is at least one selected from the group consisting of methotrexate; ralitrexed; aminopterin; pralatrexate; lometrexol; thiophene analog of lometrexol; furan analog of lometrexol; trimetrexed; LY309887; and GW 1843U89. Alternatively, or in addition, the bioactive antifolate agent is at least one selected from at least one from the group consisting of proguanil; pyrimethamine; trimethoprim and 6-Substituted Pyrrolo and Thieon[2,3-d]pyrrolopyrimidine class of GARFT inhibitors. Lometrexol analogs are described, for example, in Habeck et al., Cancer Research, v. 54, page 1021-1026, Feb 15, 1994.

The bioactive antifolate agent or any bioactive agent may be at a pH of 5-8 in the liposomal antifolate composition. Alternatively, the liposomal antifolate composition may comprise bioactive antifolate agent at a pH of 2-6.

Any of the moieties, such as the targeting moiety, the detectable label, the immunostimulatory agent, the steric stabilizer, and any optional moieties and agents may be bound to the liposome or liposomal component directly or indirectly. Indirect binding may include binding through a steric stabilizer (e.g., PEG), a functional group such as maleimide, an ionic bond (avidin, streptavidin, biotin and the like), or a binding pair (NTA-nickel and the like). Combinations of these indirect binding mechanism are also envisioned such as, for example, PEG- maleimide.

In the liposomal antifolate composition or other composition, the targeting moiety may be bound via a maleimide functional group to at least one selected from the group consisting of a liposomal component and a PEG molecule. The targeting moiety may have specific affinity for at least one selected from the group consisting of: folate receptor alpha; folate receptor beta; and folate receptor delta. For example, the targeting moiety has specific affinity for at least two selected from the group consisting of: folate receptor alpha; folate receptor beta; and folate receptor delta. As a further example, the targeting moiety may have has specific affinity for all three of folate receptor alpha; folate receptor beta; and folate receptor delta.

In an example embodiment, the targeting moiety has specific affinity for an epitope on a tumor cell surface antigen that is present on a tumor cell but absent or inaccessible on a non-tumor cell. The tumor cell may be, for example, a malignant cell. The tumor cell surface antigen can be at least one selected from the group consisting of: folate receptor alpha; folate receptor beta; and folate receptor delta. In one sample measurement of affinity, the targeting moiety may bind folate receptor with an affinity that is at least 2 folds, 5 folds, 10 folds, 25 folds, 100 folds, 500 folds or 5000 folds stronger than a binding affinity to a reduced folate carrier.

In the example embodiments which involve a targeting moiety, the targeting moiety may be a protein comprising an antigen binding sequence of an antibody. The antigen binding sequence of an antibody comprises one or more complementary determining regions of antibody origin. The protein may comprise an antibody. In an example embodiment, the targeting moiety is at least one selected from the group consisting of an antibody; a humanized antibody; an antigen binding fragment of an antibody; a single chain antibody; a single-domain antibody; a bi-specific antibody; a synthetic antibody; a pegylated antibody; and a multimeric antibody.

The liposomes of the liposomal antifolate composition or liposomal composition may comprise up to 200 or up to 250 targeting moieties per liposome. As an example, the liposome may comprise 30 to 200 targeting moieties.

One aspect is also directed to a method of delivering a bioactive antifolate agent to a tumor expressing folate receptor on its surface, the method comprising: administering any of the compositions such as the liposomal antifolate composition in an amount to deliver a therapeutically effective dose of the bioactive antifolate agent to the tumor. Administering may be selected from the group consisting of: infusion; injection; parenteral administration; and topical administration. The subject may be any animal or any mammal. Examples of suitable animals are listed in this disclosure. For example, the subject can be a human.

The compositions may be prepared using any suitable method. Methods of preparing a liposomal antifolate composition or liposomal composition according to claim 19 and claim 37 are provided. The methods comprise the steps of: forming a mixture comprising: (1) liposomal components; (2) the bioactive antifolate agent in aqueous solution; (3) the targeting moiety which optionally may be already attached or bonded to a liposomal component. The next steps involves homogenizing the mixture to form liposomes in said aqueous solution; and extruding the mixture through a membrane to form liposomes enclosing the bioactive antifolate agent in an aqueous solution. The method may comprise an optional step of removing excess bioactive antifolate agent in aqueous solution outside of the liposomes after said extruding step. The method may further comprise an optional step of lyophilizing said composition after said removing step to form a lyophilized composition. The method may include another optional step. The step is reconstituting said lyophilizing composition by dissolving said lyophilizing composition in a solvent after said lyophilizing step.

The mixture may comprise at least one selected from the group consisting of mannitol; trehalose; sorbitol; and sucrose. The one or more liposomal components further comprises a steric stabilizer. The steric stabilizer may be at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[N-(2-hydroxypropyl) methacrylamide]; amphiphilic poly-N-vinylpyrrolidones; L-amino-acid-based polymer; and polyvinyl alcohol. The PEG may have a number average molecular weight (Mn) of 200 to 5000 daltons. In the method of making a composition the solvent may be an aqueous solvent.

A targeted liposomal composition that selectively targets folate receptors according to claim 39 is provided. The example targeted liposomal composition comprises a liposome including an interior space; a bioactive agent disposed within said interior space; a steric stabilizer molecule attached to an exterior of the liposome; and a targeting moiety comprising a protein with specific affinity for at least one folate receptor, said targeting moiety attached to at least one of the steric stabilizer and the exterior of the liposome. The steric stabilizer may be at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[N-(2-hydroxypropyl) methacrylamide]; amphiphilic poly-N-vinylpyrrolidones; L-amino-acid-based polymer; and polyvinyl alcohol. For example, the PEG may have a number average molecular weight (Mn) of 200 to 5000 daltons. In this targeted liposomal composition, the bioactive agent comprises at least one of the group consisting of ellipticine; paclitaxel; pemetrexed; methotrexate; ralitrexed; aminopterin; pralatrexate; lometrexol; thiophene analog of lometrexol; furan analog of lometrexol; trimetrexed; LY309887; GW 1843U89; proguanil; pyrimethamine; trimethoprim and 6-Substituted Pyrrolo and Thieon[2,3-d]pyrrolopyrimidine class of GARFT inhibitors.

The composition is made by a method according to claim 41, by forming a mixture comprising: (1) liposomal components; (2) the bioactive agent in aqueous solution; (3) the targeting moiety. The next steps involve homogenizing the mixture to form liposomes in said aqueous solution; and extruding the mixture through a membrane to form liposomes enclosing the bioactive antifolate agent in an aqueous solution. An optional step may involve removing excess bioactive antifolate agent in aqueous solution outside of the liposomes after said extruding step. Another optional step involves lyophilizing said composition after said removing step to form a lyophilized composition. Another optional step involves reconstituting said lyophilizing composition by dissolving said lyophilizing composition in a solvent after said lyophilizing step. The other components and steps may be shared from the other method of making as discussed herein.

A kit for providing any liposomal composition, including liposomal antifolate composition according to claim 43 is also provided. The kit comprises the liposomal components, an instruction for using the composition to encapsulate a bioactive agent, and optionally, in a separate container, the bioactive agent.

### BRIEF DESCRIPTION OF THE DRAWINGS

- Figure 1A: is a schematic illustrating normal tissue.
- Figure 1B: is a schematic illustrating cancerous tissue.
- Figure 2: is a schematic illustrating and example embodiment and its binding mechanism.
- Figure 3: is a schematic illustrating a fluorochrome conjugated antibody binding to a folate receptor on a cell surface.
- Figure 4: is a schematic showing an example liposome binding to and internalizing into a cell expressing folate receptor alpha.
- Figure 5: is a schematic illustrating the effect of internalization of an example liposomal composition on cell proliferation using p38 protein kinase pathways.
- Figure 6: depicts data from flow cytometry analysis of KB cells using flurochrome.
- Figure 7: depicts data from flow cytometry analysis of OVCAR-3 (ovarian) cells using flurochrome.
- Figure 8: depicts data from flow cytometry analysis of NCIH2452 (mesothelioma) cells using flurochrome.
- Figure 9: depicts data from flow cytometry analysis of CCD841 (normal colon) cells using flurochrome.
- Figure 10: depicts data from flow cytometry analysis of SL0003 (lung) cells using flurochrome.
- Figure 11: depicts data from flow cytometry analysis of CCD841 (normal colon) cells using flurochrome.
- Figure 12: is a bar chart depicting surface levels of an example liposomal composition in normal or cancer cells.
- Figure 13: depicts data from flow cytometry analysis of ovarian cancer cells using RhodoRed.
- Figure 14: depicts data from flow cytometry analysis of KB folate receptor alpha high cells using RhodoRed.
- Figure 15: depicts data from flow cytometry analysis of normal breast cells using RhodoRed.
- Figure 16: depicts data from flow cytometry analysis of normal colon cells using RhodoRed.
- Figure 17: depicts a bar chart showing liposome concentration dependent targeting vs. untargeting liposome concentration dependent detection.
- Figure 18A: depicts data from flow cytometry analysis of untreated cells.
- Figure 18B: depicts data from flow cytometry analysis of cells treated with an example liposomal composition according to an example embodiment.
- Figure 19: depicts lung cancer cells exposed to various reagents as listed.
- Figure 20: is a line graph illustrating correlation between growth inhibition and folate receptor alpha expression.
- Figure 21: is a bar graph summarizing results and demonstrating that an example liposomal composition of an example embodiment inhibits cancer cell growth.
- Figure 22A: is a schematic depicting the cell cycle of a normal cell.
- Figure 22B: is a chart showing Propidium Iodide quantification of cells in various stages of cell cycle.
- Figure 23A: depicts data from Propidium Iodide quantification of cells that are untreated.
- Figure 23B: depicts data from Propidium Iodide quantification of cells that are treated with pemetrexed.
- Figure 24: is a bar chart showing cell cycle stasis by an example embodiment of a liposomal composition.
- Figure 25: depicts analysis of cells for Mac-1 to determine maturing neutrophils.
- Figure 26A: depicts flow cytometry data from normal cells.
- Figure 26B: depicts flow cytometry data from pemetrexed treated cells.
- Figure 27: is a bar chart depicting the number of differentiated neutrophils in pemetrexed treated and example embodiment treated samples.

### DETAILED DESCRIPTION

Antifolate drugs, as discussed above, were designed as folate mimetic molecules that work by interfering with the action of folates once inside a cell, depriving cells of the DNA precursors they need to replicate and proliferate. Because cancer cells are fast growing cells with a high demand for DNA precursors in the form of folates, they take up antifolate drugs in the same manner as folates and are as a result susceptible to the effects of antifolates. However, fast growing normal cells, such as cells that line the gastrointestinal (GI) tract and cells of the bone marrow such as, for example, neutrophils, divide rapidly as well using folates supplied primarily via RFCs. Normal cells are therefore also susceptible to the toxic effects of antifolates because the RFCs mediated transport mechanism which most antifolates are designed to use to infiltrate and kill cancer cells is the same mechanism that normal cells use to supply themselves with folates. As a result, treatment of cancers using very promising and effective antifolates has been a difficult challenge in the clinical care of patients because of the high likelihood of the treatment causing collateral damages to fast-growing normal cells, thereby causing antifolate- related severe and potentially life-threatening toxicities.

As discussed above, antifolates as a class are used for their antiproliferative effect in the treatment of cancer to inhibit cell growth and division, which causes cancer cells to die. The fast replicating cancer cells require increased amount of folates when compared to most normal cells. This led to the clinical development of antifolates as anticancer agents almost 70 years ago. However, though antifolate-based therapies were shown to be effective for cancer treatment, the clinical development of antifolates has been problematic and often derailed in view of a compelling clinical dilemma. This dilemma stems from two competing clinical dynamics. On one hand, antifolates are designed to be folate mimic molecules with most of them intended to reach cancer cells using RFCs as the preferred cross-membrane transport mechanism. On the other hand, fast renewing tissues in the body such as the bone marrow or intestinal track tissue cells are, like cancer cells, also highly folate-dependent and use also RFCs as the primary cross-membrane folate cell supply mechanism. The net result of these two clinical dynamics is that bone marrow and gastrointestinal (GI) tract cells have been the most prevalent sites of patients' life-threatening antifolate-related toxicities. Some of these toxicities have included mucositis, diarrhea, anemia, neutropenia, and low white blood counts. Such toxicities, alone or in combination, were in a number of instances blamed for patient death from antifolate-based treatment. The consequence is that to date many effective promising antifolates continue to fail during their development, not because of a lack of effectiveness against cancer cells, but instead because of patient safety concerns. The few that have managed to reach the stage of becoming medicines have limited use in clinical practice again due to safety concerns.

Antifolates as a class remain a promising treatment modality for cancer despite the associated risk of severe and even life-threatening toxicities for patients. The challenge is to figure out a way to deliver these highly effective antifolates in a manner that avoids damage to normal cells.

Prior efforts have generally focused on using RFCs to deliver an anticancer agent. However, the present inventors exploit another pathway that is especially prevalent in cancer cells involving folate receptors, including, but not limited to, for example, folate receptor alpha, folate receptor beta and/or folate receptor delta. It has been observed in cancer biology that cancer cells preferentially express folate receptor alpha in contrast to normal cells in order to efficiently uptake folates for the sustainment of their fast replication and proliferation needs. Cancer cells are very efficient at supplying themselves with folates contained in the blood stream as compared to normal cells. One way that cancer cells do this is by their overexpression of folate receptors, such as, for example, folate receptor alpha. As cancer progresses, tumor cell surface folate receptor alpha levels tend to increase, most likely due to increasing needs for folate supply.

Because of its high affinity to folate receptor alpha, folic acid was conventionally investigated as a targeting moiety for delivering anti-cancer or cytotoxic molecules to cancer cells with the intent to preferentially deliver a cytotoxic drug to cancer cells, either conjugated to a liposome containing the cytotoxic drug or conjugated to the cytotoxic drug itself. This approach has not led to improved patient safety in large part because, as recognized by the inventors, this approach fails to appreciate a key biological difference in exploiting folate pathways as an approach to deliver a cytotoxic to cancer cells while reducing and/or minimizing exposure of normal cells to the cytotoxic drug; with folic acid as the targeting ligand, normal cells were not being spared from toxicity since such a targeted drug was still being taken up by normal cells via RFCs. In other words, a targeted drug using folic acid as the targeting moiety is biologically no different than a regular untargeted antifolate because a drug of such construct binds to both folate receptor alpha and RFCs just like any other folate mimic molecule that is indiscriminately taken up by both cancer and normal cells. Therefore, using folic acid as the targeting moiety does not provide the selective delivery of cytotoxic agents to cancer cells while avoiding normal cells. Thus, with folic acid as the targeting moiety, drug related toxicity remained a concern in patient care. As a result, leading experts suggested that trying to exploit folate receptors as a means for selective targeting of cancer cell may be ineffective, guiding the efforts of those skilled in the art away from attempting to exploit folate receptors.

Targeting an antifolate to a folate receptor with a targeting moiety has not been attempted to date. Because antifolates mimic folates, one would not consider exploiting the folate pathways to deliver an antifolate in a targeted way. It would be considered redundant since the reduced folate carrier already transport folate into the cells. From this understanding, it was inherently logical to conclude that because an antifolate mimics a folate, an antifolate drug will be taken up effectively by a folate receptor by a cell and further assistance using, for example, an antibody would not be necessary. A counter-intuitive approach was taken by the current inventors. Because it was important to shield antifolates from being taken up by normal cells via RFCs in order to reduce or prevent antifolate-related toxicity, the inventors found that this goal could be achieved by, among other things, exploiting a cancer specific morphology which has been unappreciated as useful to the field of antifolate research: the loss of polarity by tumor tissue cells.

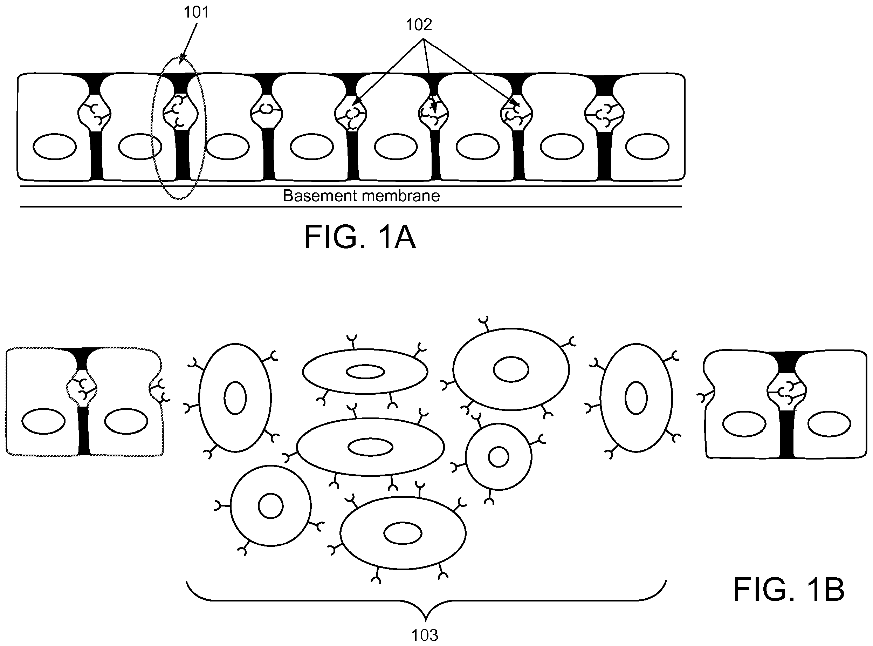

Disruption of cell polarity and tissue disorganization is a hallmark of advanced epithelial tumors. As illustrated in Figure 1A, normal simple epithelium generally comprises a monolayer of individual cells that display a distinct apical- basal polarity. Cells are tightly packed and connected to each other by the apical junctional complexes (Figure 1A-101), which separate apical and basolateral membrane domains. In normal tissue where polarity is preserved, folate receptor alpha is attached at the apical surface of cells situated away from, and out of direct contact with folates in the blood circulation (Figure 1A-102). Figure 1B illustrates how cells in high-grade epithelial tumors display loss of apical-basal polarity and overall tissue disorganization, putting folate receptor alpha in direct contact with folates in the blood circulation (1B-103). This feature of tumor tissue cells, was believed by the inventors to have greater significance for antifolate based therapies than conventional thinking had appreciated. The inventors discovered that this held a significant potential to rehabilitate antifolates as anticancer therapies while reducing and/or even minimizing associated severe and sometime life-threatening toxicities associated with antifolates.

In this regard, the inventors designed a chemical entity to deliver an antifolate agent in a manner that selectively targets folate receptors that are highly expressed in cancer cells, such as, for example, folate receptor alpha, beta and delta while avoiding RFCs (the folate pathway used by normal cells), to selectively expose the antifolate to tumor tissue cells while reducing or avoiding exposure of antifolates to normal cells. This is made possible by recognizing that following loss of polarity, tumor tissue cells not only overexpress and expose folate receptors, such as folate receptor alpha but also that folate receptors in cancer cells are in direct contact with blood circulation, both of which are not the case for the normal tissues. This approach may also extend to other cell surface folate receptors (e.g. folate receptor beta, folate receptor delta, etc.) because of their structural and functional similarities to folate receptor alpha.

The disclosure relates in general to liposome compositions useful for delivering a variety of bioactive agents, such as, for example, antifolates, methods of making the liposomal compositions and methods for treating patients using the liposomal compositions. There is special utility in providing an antifolate encapsulating liposome that is targeted to folate receptors but which is not specifically targeted to reduced folate carriers.

More specifically, the disclosure is based on the discovery that a neutral or anionic liposome (i.e., a non-cationic liposome) with affinity and specificity to a folate receptor or more than one folate receptor containing one or more bioactive agent such as, for example, an anti-cancer (antineoplastic) agent is surprisingly effective against cells presenting and expressing folate receptors on their cell surface.

In an example embodiment, a liposomal antifolate composition is provided. The liposomal antifolate composition may comprise a liposome including an interior space; a bioactive antifolate agent disposed within the interior space; a PEG molecule attached to an exterior of the liposome; and a targeting moiety comprising a protein with specific affinity for at least one folate receptor, the targeting moiety attached to at least one of the PEG and the exterior of the liposome.

The term attach or attached refers, for example, to any type of bonding such as covalent bonding, ionic bonding (e.g., avidin-biotin) bonding by hydrophobic interactions, and bonding via functional groups such as maleimide, or linkers such as PEG. For example, a detectable marker, a steric stabilizer, a liposome, a liposomal component, an immunostimulating agent may be attached to each other directly, by a maleimide functional group, or by a PEG-malemide group.

The liposomes in some example embodiments include a steric stabilizer that may increase their longevity in circulation. The basic concept is that one or more steric stabilizers such as a hydrophilic polymer (Polyethylene glycol (PEG)), a glycolipid (monosialoganglioside (GM1)) or others occupies the space immediately adjacent to the liposome surface and exclude other macromolecules from this space. Consequently, access and binding of blood plasma opsonins to the liposome surface are hindered, and thus interactions of macrophages with such liposomes, or any other clearing mechanism, are inhibited and longevity of the liposome in circulation is enhanced. In example embodiments, the steric stabilizer or the population of steric stabilizers may be a PEG or a combination comprising PEG. In an example embodiment, the steric stabilizer may be a PEG with a number average molecular weight (Mn) of 200 to 5000 daltons. These PEGs can be of any structure such as linear, branched, star or comb structure and are commercially available.

The liposomes contained in the liposome composition of various example embodiments can be any liposome known or later discovered in the art. In general, the liposomes of the example embodiments may have any liposome structure, e.g., structures having an inner space sequestered from the outer medium by one or more lipid bilayers, or any microcapsule that has a semi-permeable membrane with a lipophilic central part where the membrane sequesters an interior. A lipid bilayer can be any arrangement of amphiphilic molecules characterized by a hydrophilic part (hydrophilic moiety) and a hydrophobic part (hydrophobic moiety). Usually amphiphilic molecules in a bilayer are arranged into two dimensional sheets in which hydrophobic moieties are oriented inward the sheet while hydrophilic moieties are oriented outward. Amphiphilic molecules forming the liposomes of the example embodiments can be any known or later discovered amphiphilic molecules, e.g., lipids of synthetic or natural origin or biocompatible lipids. Liposomes of the example embodiments may also be formed by amphiphilic polymers and surfactants, e.g., polymerosomes and niosomes. For the purpose of this disclosure, without limitation, these liposome-forming materials also are referred to as "lipids".

The liposome composition may be a liquid or it may be dry, such as, for example, in the form of a dry powder or a dry cake. The dry powder or dry cake may have undergone primary drying under, for example, lyophilization conditions or optionally, it may have undergone both primary drying only or both primary drying and secondary drying. In the dry form, the powder or cake may, for example, have between 1% to 6% moisture, for example, such as between 2% to 5% moisture or between 2% to 4% moisture. One example method of drying is lyophilization (also called freeze-drying, or cyrodessication). Any of the compositions and methods of the disclosure may involve the liposomes, lyophilized liposomes or liposomes reconstituted from lyophilized liposomes. In lyophilization, lyoprotectants or cryoprotectants, molecules protect freeze-dried material may be used. These molecules are typically polyhydroxy compounds such as sugars (mono-, di-, and polysaccharides), polyalcohols, and their derivatives, glycerol, or polyethyleneglycol, trehalose, maltose, sucrose, glucose, lactose, dextran, glycerol, and aminoglycosides. The lyoprotectants or cryoprotectants may, for example, comprise up to 10% or up to 20% of a solution outside the liposome or inside the liposome or both outside and inside the liposome.

The liposomes of the example embodiments may, for example, have a diameter of in the range of 30-150 nm (nanometer). In other example embodiments, the liposome may, for example, have a diameter in the range of 40-70 nm.

The liposomes of the example embodiments are anionic or neutral. That is, the liposome should not be cationic. The determination of the charge (i.e., anionic, neutral or cationic) may be made by measuring the zeta potential of the liposome. In an embodiment according to the invention, the zeta potential of the liposome is less than or equal to zero. In another example embodiment, the zeta potential of the liposome is in a range of 0 to -150 mV. In another example embodiment, the zeta potential should be in the range of -30 to -50 mV.

The properties of liposomes are influenced by the nature of lipids used to make the liposomes. A wide variety of lipids have been used to make liposomes. These include cationic, anionic and neutral lipids. Cationic lipids are used to make cationic liposomes which are commonly used as gene transfection agents. The positive charge on cationic liposomes enables interaction with the negative charge on cell surfaces. Following binding of the cationic liposomes to the cell, the liposome is transported inside the cell through endocytosis. However, cationic liposomes will bind to both normal cells and tumor cells. Because the example embodiments are intended to specifically and selectively target tumor cells while substantially sparing normal cells, the use of cationic lipids is not preferred. Using a mixture of, for example, neutral lipids such as HSPC and anionic lipids such as PEG-DSPE results in the formation of anionic liposomes which are less likely to non-specifically bind to normal cells. Specific binding to tumor cells can be achieved by using a tumor targeting antibody such as, for example, a folate receptor antibody, including, for example, folate receptor alpha antibody, folate receptor beta antibody and/or folate receptor delta antibody.

As an example, at least one (or some) of the lipids is/are amphipathic lipids, defined as having a hydrophilic and a hydrophobic portions (typically a hydrophilic head and a hydrophobic tail). The hydrophobic portion typically orients into a hydrophobic phase (e.g., within the bilayer), while the hydrophilic portion typically orients toward the aqueous phase (e.g., outside the bilayer). The hydrophilic portion may comprise polar or charged groups such as carbohydrates, phosphate, carboxylic, sulfato, amino, sulfhydryl, nitro, hydroxy and other like groups. The hydrophobic portion may comprise apolar groups that include without limitation long chain saturated and unsaturated aliphatic hydrocarbon groups and groups substituted by one or more aromatic, cyclo-aliphatic or heterocyclic group(s). Examples of amphipathic compounds include, but are not limited to, phospholipids, aminolipids and sphingolipids.

Typically, for example, the lipids are phospholipids. Phospholipids include without limitation phosphatidylcholine, phosphatidylethanolamine, phosphatidylglycerol, phosphatidylinositol, phosphatidylserine, and the like. It is to be understood that other lipid membrane components, such as cholesterol, sphingomyelin, cardiolipin, etc. may be used.

In an example embodiment, the lipids may be anionic and neutral (including zwitterionic and polar) lipids including anionic and neutral phospholipids. Neutral lipids exist in an uncharged or neutral zwitterionic form at a selected pH. At physiological pH, such lipids include, for example, dioleoylphosphatidylglycerol (DOPG), diacylphosphatidylcholine, diacylphosphatidylethanolamine, ceramide, sphingomyelin, cephalin, cholesterol, cerebrosides and diacylglycerols. Examples of zwitterionic lipids include without limitation dioleoylphosphatidylcholine (DOPC), dimyristoylphosphatidylcholine (DMPC), and dioleoylphosphatidylserine (DOPS). An anionic lipid is a lipid that is negatively charged at physiological pH. These lipids include without limitation phosphatidylglycerol, cardiolipin, diacylphosphatidylserine, diacylphosphatidic acid, N-dode- canoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

Collectively, anionic and neutral lipids are referred to herein as non-cationic lipids. Such lipids may contain phosphorus but they are not so limited. Examples of non-cationic lipids include lecithin, lysolecithin, phosphatidylethanolamine, lysophosphatidylethanolamine, dioleoylphosphati- dylethanolamine (DOPE), dipalmitoyl phosphatidyl ethanolamine (DPPE), dimyristoylphosphoethanolamine (DMPE), distearoyl-phosphatidy 1-ethanolamine (DSPE), palmitoyloleoyl-phosphatidylethanolamine (POPE) palmitoyloleoylphosphatidylcholine (POPC), egg phosphatidylcholine (EPC), distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), palmitoyloleyolphosphatidylglycerol (POPG), 16-0-monomethyl PE, 16-0- dimethyl PE, 18-1-trans PE, palmitoyloleoyl-phosphatidylethanolamine (POPE), 1-stearoyl-2-oleoylphosphatidyethanolamine (SOPE), phosphatidylserine, phosphatidylinositol, sphingomyelin, cephalin, cardiolipin, phosphatidic acid, cerebrosides, dicetylphosphate, and cholesterol.

Liposomes of example embodiments may be assembled using any liposomal assembly method using liposomal components (also referred to as liposome components). Liposomal components include, for example, lipids such as DSPE, HSPC, cholesterol and derivatives of these components. Other suitable lipids are commercially available for example, by Avanti Polar Lipids, Inc. (Alabaster, Alabama, U.S.A.). A partial listing of available negatively or neutrally charged lipids suitable for making anionic liposomes, may be, for example, at least one of the following: DLPC, DMPC, DPPC, DSPC, DOPC, DMPE, DPPE, DOPE, DMPA•Na, DPPA•Na, DOPA•Na, DMPG•Na, DPPG•Na, DOPG•Na, DMPS•Na, DPPS•Na, DOPS•Na, DOPE-Glutaryl•(Na)₂, Tetramyristoyl Cardiolipin•(Na)₂, DSPE-mPEG-2000•Na, DSPE-mPEG-5000•Na, and DSPE-Maleimide PEG-2000•Na.

Derivatives of these lipids may, for example, include, at least, the bonding (preferably covalent bonding) of one or more steric stabilizers and/or functional groups to the liposomal component after which the steric stabilizers and/or functional groups should be considered part of the liposomal components. Functional groups comprises groups that can be used to attach a liposomal component to another moiety such as a protein. Such functional groups include, at least, maleimide. These steric stabilizers include at least one from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[N-(2-hydroxypropyl) methacrylamide]; amphiphilic poly-N-vinylpyrrolidones; L-amino-acid-based polymer; and polyvinyl alcohol.

Because a liposomal components may include any molecule(s) (i.e., chemical/reagent/protein) that is bound to it, the liposomal components may, for example, include, at least, DSPE, DSPE-PEG, DSPE-maleimide, HSPC; HSPC-PEG; HSPC-maleimide; cholesterol; cholesterol-PEG; and cholesterol-maleimide. In a preferred embodiment, the liposomal components that make up the liposome comprises DSPE; DSPE-FITC; DSPE-maleimide; cholesterol; and HSPC.

In an example embodiment, at least one component of the lipid bilayer is functionalized (or reactive). As used herein, a functionalized component is a component that comprises a reactive group that can be used to crosslink reagents and moieties to the lipid. If the lipid is functionalized, any liposome that it forms is also functionalized.

In example embodiments, the reactive group is one that will react with a crosslinker (or other moiety) to form crosslinks. The reactive group may be located anywhere on the lipid that allows it to contact a crosslinker and be crosslinked to another moiety (i.e., steric stabilizer, targeting moiety, etc.). In some embodiments, it is in the head group of the lipid, including for example a phospholipid. An example of a reactive group is a maleimide group. Maleimide groups may be crosslinked to each other in the presence of dithiol crosslinkers such as but not limited to dithiolthrietol (DTT).

It is to be understood that the example embodiments contemplate the use of other functionalized lipids, other reactive groups, and other crosslinkers. In addition to the maleimide groups, other examples of reactive groups include but are not limited to other thiol reactive groups, amino groups such as primary and secondary amines, carboxyl groups, hydroxyl groups, aldehyde groups, alkyne groups, azide groups, carbonyls, halo acetyl (e.g., iodoacetyl) groups, imidoester groups, N-hydroxysuccinimide esters, sulfhydryl groups, pyridyl disulfide groups, and the like.

Functionalized and non-functionalized lipids are available from a number of commercial sources including Avanti Polar 5 Lipids (Alabaster, Ala.).

The liposomes of example embodiments may further comprise an immunostimulatory agent, a detectable marker, or both disposed on its exterior. For example, immunostimulatory agent or detectable marker may be ionically bonded or covalently bonded to an exterior of the liposome, including, for example, optionally to the steric stabilizer.

Immunostimulatory agents, also known as immunostimulants, immunostimulators, haptens and adjuvants, are substances that stimulate the immune system by inducing activation or increasing activity of any of its components.

These immunostimulatory agents can include one or more of a hapten, an adjuvant, a protein immunostimulating agent, a nucleic acid immunostimulating agent, and a chemical immunostimulating agent. Many adjuvants contain a substance designed to stimulate immune responses, such as lipid A, Bortadella pertussis or Mycobacterium tuberculosis derived proteins. Certain adjuvants are commercially available as, for example, Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants. In a preferred embodiment, the immunostimulant may be at least one selected from the group consisting of fluorescein, DNP, beta glucan, beta-1,3-glucan, beta-1,6-glucan.

A detectable marker may, for example, include, at least, a radioisotope, a fluorescent compound, a bioluminescent compound, chemiluminescent compound, a metal chelator, an enzyme, a dye, an ink, a magnetic compound, a biocatalyst or a pigment that is detectable by any suitable means known in the art, e.g., magnetic resonance imaging (MRI), optical imaging, fluorescent/luminescent imaging, or nuclear imaging techniques.

The immunostimulatory agent and/or detectable marker may be attached to the exterior by co-incubating it with the liposome. For example, the immunostimulatory agent and/or detectable marker may be associated with the liposomal membrane by hydrophobic interactions or by an ionic bond such as an avidin/biotin bond or a metal chelation bond (e.g., Ni-NTA). Alternatively, the immunostimulatory agent or detectable marker may be covalently bonded to the exterior of the liposome such as, for example, by being covalently bonded to a liposomal component or to the steric stabilizer which is the PEG.

One example reagent is fluorescein isothiocyanate (FITC) which, based on our experiments, may surprisingly serve as both an immunostimulant and a detectable marker.

Example embodiments also provide for a liposome that encloses an interior space. In an example embodiment, the interior space may comprise, but is not limited to, an aqueous solution. The interior space may comprise a bioactive agent, such as, for example, an antifolate agent and an aqueous pharmaceutically acceptable carrier. The pharmaceutically acceptable carrier may comprise, for example, trehalose. In an example embodiment, the trehalose may, for example, be present at about 5% to 20% weight percent of trehalose or any combination of one or more lyoprotectants or cryoprotectants at a total concentration of 5% to 20%. The interior space may, for example, comprise a citrate buffer at a concentration of between 5 to 200 mM. The citrate buffer may buffer the interior space at a pH of between 2.8 to 6. Independent of the trehalose or citrate concentration, the pharmaceutically acceptable carrier may comprise a total concentration of sodium acetate and calcium acetate of between 50 mM to 500 mM

In an example embodiment, the bioactive antifolate agent may, for example, be a water soluble bioactive agent. That is, the bioactive agent may form an aqueous solution. According to example embodiments, each liposome may comprise an interior space that contains less than 200,000 molecules of the bioactive agent. For example, in an example embodiment, the liposome may comprise between 10,000 to 100,000 of a bioactive antifolate agent.

In an example embodiment, the bioactive agent can be at least one from the group consisting of pemetrexed, lometrexol, methotrexate, ralitrexed, aminopterin, pralatrexate, lometrexol analogs thereof, thiophene analog of lometrexol, furan analog of lometrexol, trimetrexed, LY309887; and GW 1843U89. In another embodiment, the bioactive agent can be at least one from the group consisting of proguanil, pyrimethamine, trimethoprim and 6-Substituted Pyrrolo and Thieon[2,3-d]pyrrolopyrimidine class of GARFT inhibitors. In one preferred embodiment, the bioactive antifolate agent is pemetrexed. In another example embodiment, the bioactive antifolate agent is lometrexol.

The pH of a solution comprising the bioactive agent may, for example, be set, for example, to from 5 to 8 or from 2 to 6.

According to the example embodiments, the liposomes contained in the liposome composition of the examples can also be targeting liposomes, e.g., liposomes including one or more targeting moieties or biodistribution modifiers on the surface of the liposomes. Example embodiments of targeting liposomes may, for example, be called immunoliposomes. A targeting moiety can be any agent that is capable of specifically binding or interacting with a desired target. In an example embodiment, a targeting moiety may be a moiety that binds with specificity and affinity to a folate receptor, such as, for example, folate receptor alpha, folate receptor beta and/or folate receptor delta. Folate receptors are distinct and different from reduced folate carriers and exploit different pathways to the interior of the cells. The targeting moiety, according to example embodiments, specifically and preferentially binds to and/or internalizes into, a target cell in which the liposome-entrapped entity exerts its desired effect. A target cell may, for example, be a cancer cell, a tumor cell and/or a metastatic cell. In an example embodiment, the liposome carrying a targeting moiety is internalized by a target cell.

In any of the example embodiments of this disclosure, the targeting moiety may be a protein which an antigen binding sequence of an antibody. In an example embodiment, the protein may, for example, have a three-dimensional structure of, at least, the antigen binding site of an antibody. One example of such a protein as a targeting moiety is an antibody. However a complete antibody is not necessary. For example, a protein which is a targeting moiety of any of the example embodiments may comprise one or more complementary determining regions (CDRs) of antibody origin. Examples of suitable proteins that can serve as targeting moieties include at least one selected from the group consisting of an antibody, a humanized antibody, an antigen binding fragment of an antibody, a single chain antibody, a single-domain antibody, a bi-specific antibody, a synthetic antibody, a pegylated antibody and a multimeric antibody. An antibody may have a combination of these characteristics. For example, a humanized antibody may be an antigen binding fragment and may be pegylated and multimerized as well. Antibodies to folate receptor alpha are commercially available.

An example antibody that may be employed is a murine antibody against folate receptor alpha. The sequence is described in U.S. patent US5646253. For example, based on the sequences disclosed, the gene was synthesized and placed into a transient expression vector and the antibody was produced in HEK-293 transient expression system. The antibody can be a complete antibody, a Fab, or any of the various antibody variations discussed.

Each of the liposomes may comprise, for example from 30 to 250 targeting moieties, such as, for example, from 30-200 targeting moieties. Alternatively, each of the liposomes may comprise less than 220 targeting moieties such as, for example, less than 200 moieties. The targeting moieties can be attached, such as, for example, by being covalently bonded to the outside of the liposome. The molecules that are on the outside of the liposome may, for example, comprise, at least, a lipid, a steric stabilizer, a maleimide, a cholesterol and the like. In an example embodiment, the targeting moiety may be covalently bound via a maleimide functional group to at least one selected from the group consisting of a liposomal component and a steric stabilizer such as a PEG molecule. It is possible that all the targeting moieties are bound to one component such as PEG. It is also possible that the targeting moieties are bound to different components. For example, some targeting moieties may be bound to the lipid components or cholesterol, some targeting moieties may be bound to the steric stabilizer (e.g., PEG) and still other targeting moieties may be bound to a detectable marker or to another targeting moiety.

In an example embodiment, the targeting moiety has affinity and specificity for at least one or more antigen where the antigen is selected from the group consisting of folate receptor alpha, folate receptor beta, and folate receptor delta. In an example embodiment, the targeting moiety has specific affinity (i.e., affinity and specificity) for at least two antigens selected from the group consisting of folate receptor alpha, folate receptor beta, and folate receptor delta. In another example embodiment, the targeting moiety has specific affinity for three antigens which are, for example, folate receptor alpha; folate receptor beta; and folate receptor delta. The targeting moiety may have affinity and specificity to an epitope of the antigen because sometimes a targeting moiety does not bind the complete antigen but just an epitope of many epitopes in an antigen. In an example embodiment, the targeting moiety has specific affinity for an epitope on a tumor cell surface antigen that is present on a tumor cell but absent or inaccessible on a non-tumor cell. For example, in some situations, the tumor antigen may be on the surface of both normal cells and malignant cancer cells but the tumor epitope may only be exposed in a cancer cell. As a further example, a tumor antigen may experience a confirmation change in cancer causing cancer cell specific epitopes to be present. A targeting moiety with specific affinity to epitopes described above are useful and envisioned in the example embodiments. In these embodiments, the tumor cell with cancer cell specific epitopes may be a cancer cell. Examples of such tumor cell surface antigens include, at least, folate receptor alpha, folate receptor beta and folate receptor delta.

Example embodiments relate to a liposomal antifolate composition comprising: a medium comprising a liposome including an interior space; an aqueous bioactive antifolate agent disposed within said interior space; a targeting moiety comprising a protein with specific affinity for at least one folate receptor, said targeting moiety disposed at an the exterior of the liposome. In the example embodiments, the medium is an aqueous solution. In an example embodiment, the interior space, the exterior space (i.e., the medium), or both the interior space and the medium contains one or more lyoprotectants or cryoprotectants which are listed above. In an example embodiment, the cryoprotectants mannitol, trehalose, sorbitol, and sucrose are preferred.

As discussed above, the liposomes of example embodiments may comprise a steric stabilizer that can increase their longevity in circulation. The basic concept is that one or more steric stabilizers such as a hydrophilic polymer (Polyethylene glycol (PEG)), a glycolipid (monosialoganglioside (GM1)) or others occupies the space immediately adjacent to the liposome surface and exclude other macromolecules from this space. Consequently, access and binding of blood plasma opsonins to the liposome surface are hindered, and thus interactions of macrophages with such liposomes, or any other clearing mechanism, are inhibited and longevity of the liposome in circulation is enhanced.

For any of the example embodiments which incorporate a steric stabilizer, the steric stabilizer may be at least one from the group consisting of polyethylene glycol (PEG), poly-L-lysine (PLL), monosialoganglioside (GM1), poly(vinyl pyrrolidone) (PVP), poly(acrylamide) (PAA), poly(2-methyl-2-oxazoline), poly(2-ethyl-2-oxazoline), phosphatidyl polyglycerol, poly[N-(2-hydroxypropyl) methacrylamide], amphiphilic poly-N-vinylpyrrolidones, L-amino-acid-based polymer, and polyvinyl alcohol. In example embodiments, the steric stabilizer or the population of steric stabilizer is PEG. In an example embodiment, the steric stabilizer is a PEG with a number average molecular weight (Mn) of 200 to 5000 daltons. These PEGs can be of any structure such as linear, branched, star or comb structure and are commercially available.

According to example embodiments, the liposome composition may be provided as a pharmaceutical composition containing the example liposome composition of the example embodiments and a carrier, e.g., pharmaceutically acceptable carrier. Examples of pharmaceutically acceptable carries are normal saline, isotonic dextrose, isotonic sucrose, Ringer's solution, and Hanks' solution. A buffer substance can be added to provide pH optimal for storage stability. For example, pH between about 6.0 and about 7.5, more preferably pH about 6.5, is optimal for the stability of liposome membrane lipids, and provides for excellent retention of the entrapped entities. Histidine, hydroxyethylpiperazine-ethylsulfonate (HEPES), morpholipoethylsulfonate (MES), succinate, tartrate, and citrate, typically at 2-20 mM concentration, are exemplary buffer substances. Other suitable carriers include, e.g., water, buffered aqueous solution, 0.4% NaCl, 0.3% glycine, and the like. Protein, carbohydrate, or polymeric stabilizers and tonicity adjusters can be added, e.g., gelatin, albumin, dextran, or polyvinylpyrrolidone. The tonicity of the composition can be adjusted to the physiological level of 0.25-0.35 mol/kg with glucose or a more inert compound such as lactose, sucrose, mannitol, or dextrin. These compositions may be sterilized by conventional, well known sterilization techniques, e.g., by filtration. The resulting aqueous solutions may be packaged for use or filtered under aseptic conditions and lyophilized, the lyophilized preparation being combined with a sterile aqueous medium prior to administration.

The pharmaceutical liposome compositions can also contain other pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. Additionally, the liposome suspension may include lipid-protective agents which protect lipids against free-radical and lipid-peroxidative damages on storage. Lipophilic free-radical quenchers, such as alpha-tocopherol and water-soluble iron-specific chelators, such as ferrioxamine, are suitable.

The concentration of the liposomes of example embodiments in the fluid pharmaceutical formulations can vary widely, i.e., from less than about 0.05% usually or at least about 2-10% to as much as 30 to 50% by weight and will be selected primarily by fluid volumes, viscosities, etc., in accordance with the particular mode of administration selected. For example, the concentration may be increased to lower the fluid load associated with treatment. This may be particularly desirable in patients having atherosclerosis-associated congestive heart failure or severe hypertension. Alternatively, liposome pharmaceutical compositions composed of irritating lipids may be diluted to low concentrations to lessen inflammation at the site of administration.

Example embodiments relate to a method of delivering a bioactive agent, such as, for example, an antifolate, to a tumor expressing folate receptor on its surface. An example method comprises the step of administering at least one of any of the compositions comprising a liposome in this disclosure in an amount to deliver a therapeutically effective dose of the bioactive antifolate agent to the tumor.

The amount of liposome pharmaceutical composition administered will depend upon the particular therapeutic entity entrapped inside the liposomes, the disease state being treated, the type of liposomes being used, and the judgment of the clinician. Generally the amount of liposome pharmaceutical composition administered will be sufficient to deliver a therapeutically effective dose of the particular therapeutic entity.

The quantity of liposome pharmaceutical composition necessary to deliver a therapeutically effective dose can be determined by routine in vitro and in vivo methods, common in the art of drug testing. See, for example, D. B. Budman, A. H. Calvert, E. K. Rowinsky (editors). Handbook of Anticancer Drug Development, LWW, 2003. Therapeutically effective dosages for various therapeutic entities are well known to those of skill in the art; and according to the example embodiments a therapeutic entity delivered via the pharmaceutical liposome composition and provides at least the same or higher activity than the activity obtained by administering the same amount of the therapeutic entity in its routine non-liposome formulation. Typically the dosages for the liposome pharmaceutical composition of the example embodiments may, for example, range between about 0.005 and about 500 mg of the therapeutic entity per kilogram of body weight, most often, between about 0.1 and about 100 mg therapeutic entity/kg of body weight.

An effective amount is a dosage of the agent sufficient to provide a medically desirable result. The effective amount will vary with the desired outcome, the particular condition being treated or prevented, the age and physical condition of the subject being treated, the severity of the condition, the duration of the treatment, the nature of the concurrent or combination therapy (if any), the specific route of administration and like factors within the knowledge and expertise of the health practitioner. It is preferred generally that a maximum dose be used, that is, the highest safe dose according to sound medical judgment.

For example, if the subject has a tumor, an effective amount may be that amount that reduces the tumor volume or load (as for example determined by imaging the tumor). Effective amounts may also be assessed by the presence and/or frequency of cancer cells in the blood or other body fluid or tissue (e.g., a biopsy). If the tumor is impacting the normal functioning of a tissue or organ, then the effective amount may be assessed by measuring the normal functioning of the tissue or organ. In some instances the effective amount is the amount required to lessen or eliminate one or more, and preferably all, symptoms.

The example embodiments provide pharmaceutical compositions. Pharmaceutical compositions are sterile compositions that comprise a sample liposome and preferably antifolate agent(s), preferably in a pharmaceutically-acceptable carrier.

The term "pharmaceutically-acceptable carrier" may, for example, refer to one or more compatible solid or liquid filler, diluents or encapsulating substances which are suitable for administration to a human or other subject contemplated by the example embodiments.

The term "carrier" denotes an organic or inorganic ingredient, natural or synthetic, with which liposome compositions are combined to facilitate administration. The components of the pharmaceutical compositions are comingled in a manner that precludes interaction that would substantially impair their desired pharmaceutical efficiency. Suitable buffering agents include acetic acid and a salt (1-2% W/V); citric acid and a salt (1-3% W/V); boric acid and a salt (0.5-2.5% W/V); and phosphoric acid and a salt (0.8-2% W/V). Suitable preservatives include benzalkonium chloride (0.003-0.03% W/V); chlorobutanol (0.3-0.9% W/V); and parabens (0.01-0.25% W/V).

Unless otherwise stated herein, a variety of administration routes are available. The particular mode selected will depend, of course, upon the particular active agent selected, the particular condition being treated and the dosage required for therapeutic efficacy. The methods provided, generally speaking, may be practiced using any mode of administration that is medically acceptable, meaning any mode that produces effective levels of a desired response without causing clinically unacceptable adverse effects. Possible administration routes include injections, by parenteral routes such as intramuscular, subcutaneous, intravenous, intraarterial, intraperitoneal, intraarticular, intraepidural, intrathecal, intravenous, intramuscular, intra sternal injection or infusion or others, as well as oral, nasal, mucosal, sublingual, intratracheal, ophthalmic, rectal, vaginal, ocular, topical, transdermal, pulmonary, inhalation.

In an example embodiment, the liposome pharmaceutical composition may, for example, be prepared as an infusion composition, an injection composition, a parenteral composition, or a topical composition, either as a liquid solution or suspension. However, solid forms suitable for solution in, or suspension in, liquid vehicles prior to injection may also be prepared. The composition may, for example, also be formulated into an enteric-coated tablet or gel capsule according to known methods in the art.

For the delivery of liposomal drugs formulated according to example embodiments, to tumors of the central nervous system, a slow, sustained intracranial infusion of the liposomes directly into the tumor (a convection-enhanced delivery, or CED) may be of particular advantage. See Saito, et al., Cancer Research, vol. 64, p. 2572-2579, 2004; Mamot, et al., J. Neuro-Oncology, vol. 68, p. 1-9, 2004. The compositions may, for example, also be directly applied to tissue surfaces. Sustained release, pH dependent release, or other specific chemical or environmental condition mediated release administration is also specifically included in the example embodiments, e.g., by such means as depot injections, or erodible implants. A few specific examples are listed below for illustration.

For oral administration, the compounds may, for example, be formulated readily by combining the liposomal compositions with pharmaceutically acceptable carriers well known in the art. Such carriers enable formulation as tablets, pills, dragees, capsules, liquids, gels, syrups, slurries, films, suspensions and the like, for oral ingestion by a subject to be treated. Suitable excipients may, for example, include, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). Optionally the oral formulations may also be formulated in saline or buffers for neutralizing internal acid conditions or may be administered without any carriers.

Pharmaceutical preparations which can be used orally include push-fit capsules made of gelatin, as well as soft, sealed capsules made of gelatin and a plasticizer, such as glycerol or sorbitol. The push-fit capsules can contain the liposomal composition suspended in suitable liquids, such as aqueous solutions, buffered solutions, fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compositions may be conveniently delivered in the form of an aerosol spray presentation from pressurized packs or a nebulizer, with the use of a suitable propellant, e.g., dichlorodifluoromethane, trichlorofluoromethane, ichlorotetrafluoroethane, carbon dioxide or other suitable gas. In the case of a pressurized aerosol the dosage unit may be determined by providing a valve to deliver a metered amount.

When it is desirable to deliver the compositions systemically, they may be formulated for parenteral administration by injection, e.g., by bolus injection or continuous infusion. Formulations for injection may be presented in unit dosage form, e.g., in ampoules or in multidose containers. Pharmaceutical parenteral formulations include aqueous solutions of the ingredients. Aqueous injection suspensions may contain substances which increase the viscosity of the suspension, such as sodium carboxymethyl cellulose, sorbitol, or dextran. Alternatively, suspensions of liposomes may be prepared as oil-based suspensions. Suitable lipophilic solvents or vehicles include fatty oils such as sesame oil, or synthetic fatty acid esters, such as ethyl oleate or triglycerides.

Alternatively, the liposomal compositions may be in powder form or lyophilized form for constitution with a suitable vehicle, e.g., sterile pyrogen-free water, before use.

The compositions may also be formulated in rectal or vaginal compositions such as suppositories or retention enemas, e.g., containing conventional suppository bases such as cocoa butter or other glycerides.

The example embodiments contemplate administration of agents to subjects having or at risk of developing a cancer including for example a solid tumor cancer, using the compositions and liposomes of example embodiments. In an example embodiment, the cancer may, for example, be distinguished by the expression of folate receptors on its cell surface. The folate receptor may, for example, include folate receptor alpha, folate receptor beta or folate receptor delta. The example embodiments contemplate that the compositions are able to deliver higher quantities of the bioactive agents, alone or in combination, to these subjects without excessive delivery to normal cells (i.e., cells not expressing folate receptors).

Any cancers that express folate receptors may be treated. It should be noted that some cancers may express folate receptors in an early stage while the majority of cancers may express folate receptors at late stages. The cancer may be carcinoma, sarcoma or melanoma. Carcinomas include without limitation to basal cell carcinoma, biliary tract cancer, bladder cancer, breast cancer, cervical cancer, choriocarcinoma, CNS cancer, colon and rectum cancer, kidney or renal cell cancer, larynx cancer, liver cancer, small cell lung cancer, non-small cell lung cancer (NSCLC, including adenocarcinoma, giant (or oat) cell carcinoma, and squamous cell carcinoma), oral cavity cancer, ovarian cancer, pancreatic cancer, prostate cancer, skin cancer (including basal cell cancer and squamous cell cancer), stomach cancer, testicular cancer, thyroid cancer, uterine cancer, rectal cancer, cancer of the respiratory system, and cancer of the urinary system.

Sarcomas are mesenchymal neoplasms that arise in bone (osteosarcomas) and soft tissues (fibrosarcomas). Sarcomas include without limitation liposarcomas (including myxoid liposarcomas and pleiomorphic liposarcomas), leiomyosarcomas, rhabdomyosarcomas, malignant peripheral nerve sheath tumors (also called malignant schwannomas, neurofibrosarcomas, or neurogenic sarcomas), Ewing's tumors (including Ewing's sarcoma of bone, extraskeletal (i.e., not bone) Ewing's sarcoma, and primitive neuroectodermal tumor), synovial sarcoma, angiosarcomas, hemangiosarcomas, lymphangiosarcomas, Kaposi's sarcoma, hemangioendothelioma, desmoid tumor (also called aggressive fibromatosis), dermatofibrosarcoma protuberans (DFSP), malignant fibrous histiocytoma (MFH), hemangiopericytoma, malignant mesenchymoma, alveolar soft-part sarcoma, epithelioid sarcoma, clear cell sarcoma, desmoplastic small cell tumor, gastrointestinal stromal tumor (GIST), and chondrosarcoma.

Melanomas are tumors arising from the melanocytic system of the skin and other organs. Examples of melanoma include without limitation lentigomaligna melanoma, superficial spreading melanoma, nodular melanoma, and acral lentiginous melanoma.

The cancer may be a solid tumor lymphoma. Examples include Hodgkin's lymphoma, Non-Hodgkin's lymphoma, and B cell lymphoma.

The cancer may be without limitation bone cancer, brain cancer, breast cancer, colorectal cancer, connective tissue cancer, cancer of the digestive system, endometrial cancer, esophageal cancer, eye cancer, cancer of the head and neck, gastric cancer, intra-epithelial neoplasm, melanoma neuroblastoma, Non-Hodgkin's lymphoma, non-small cell lung cancer, prostate cancer, retinoblastoma, or rhabdomyosarcoma.

The example embodiments may be practiced in any subject that is likely to benefit from delivery of agents as contemplated herein. Human subjects are preferred subjects in example embodiments but subjects may also include animals such as household pets (e.g., dogs, cats, rabbits, ferrets, etc.), livestock or farm animals (e.g., cows, pigs, sheep, chickens and other poultry), horses such as thoroughbred horses, laboratory animals (e.g., mice, rats, rabbits, etc.), mammal and the like. Subjects also include fish and other aquatic species.

The subjects to whom the agents are delivered may be normal subjects. Alternatively they may have or may be at risk of developing a condition that can be diagnosed or that can benefit from delivery of one or more particular agents. In an example embodiment, such conditions include cancer (e.g., solid tumor cancers or non-solid cancer such as leukemias). In a more preferred embodiment, these conditions include cancers involving cells that express folate receptors on their cell surface.

Tests for diagnosing the conditions embraced by the example embodiments are known in the art and will be familiar to the ordinary medical practitioner. The determination of whether a cell type expresses folate receptors can be made using commercially available antibodies. These laboratory tests include without limitation microscopic analyses, cultivation dependent tests (such as cultures), and nucleic acid detection tests. These include wet mounts, stain-enhanced microscopy, immune microscopy (e.g., FISH), hybridization microscopy, particle agglutination, enzyme-linked immunosorbent assays, urine screening tests, DNA probe hybridization, serologic tests, etc. The medical practitioner will generally also take a full history and conduct a complete physical examination in addition to running the laboratory tests listed above.

A subject having a cancer may, for example, be a subject that has detectable cancer cells. A subject at risk of developing a cancer may, for example, be a subject that has a higher than normal probability of developing cancer. These subjects include, for instance, subjects having a genetic abnormality that has been demonstrated to be associated with a higher likelihood of developing a cancer, subjects having a familial disposition to cancer, subjects exposed to cancer causing agents (i.e., carcinogens) such as tobacco, asbestos, or other chemical toxins, and subjects previously treated for cancer and in apparent remission.

In an example embodiment, the methods may selectively deliver a liposomal antifolate composition to the tumor at a rate which is higher, e.g. at least two-fold greater, than a cell not expressing folate receptor.

Example embodiments relate to a method of making any of the compositions of this disclosure. In an example embodiment, the method involves forming a mixture comprising: (1) liposomal components; (2) the bioactive antifolate agent in aqueous solution; and (3) the targeting moiety. The mixture may then be homogenized to form liposomes in said aqueous solution. Further, the mixture may be extruded through a membrane to form liposomes enclosing the bioactive antifolate agent in an aqueous solution. It is understood the liposomal components comprise any lipid (including cholesterol) of this disclosure including functionalized lipids and lipids attached to targeting moieties, detectable labels, and steric stabilizers, or any subset of all of these. It is further noted that the bioactive antifolate in aqueous solution may comprise any reagents and chemicals discussed for the interior or exterior of the liposome including, for example, buffers, salts, cryoprotectants and the like.

The method may further comprise the optional step of lyophilizing the composition after said removing step to form a lyophilized composition. As stated above, the bioactive antifolate agent in aqueous solution may comprise cryoprotectants which may be any cryoprotectants are listed in this disclosure. If the composition is to be lyophilized, a cryoprotectant may be preferred.

Further, after the lyophilizing step, the method can comprise the optional step of reconstituting said lyophilized composition by dissolving the lyophilized composition in a solvent after said lyophilizing step. Methods of reconstitution are well known. One preferred solvent is water. Other preferred solvents include saline solutions and buffered solutions.

While certain example embodiments are discussed, it should be understood that liposomes can be made by any method that is known or will become known in the art. See, for example, G. Gregoriadis (editor), Liposome Technology, vol. 1-3, 1st edition, 1983; 2nd edition, 1993, CRC Press, 45 Boca Raton, Fla. Examples of methods suitable for making liposome composition include extrusion, reverse phase evaporation, sonication, solvent (e.g., ethanol) injection, microfluidization, detergent dialysis, ether injection, and dehydration/rehydration. The size of liposomes can be controlled by controlling the pore size of membranes used for low pressure extrusions or the pressure and number of passes utilized in microfluidisation or any other suitable methods.

In general, the bioactive antifolate agent is contained inside, that is, in the inner (interior) space of the liposomes. In an example embodiment, the substituted ammonium is partially or substantially completely removed from the outer medium surrounding the liposomes. Such removal can be accomplished by any suitable means known to one skilled in the art, e.g., dilution, ion exchange chromatography, size exclusion chromatography, dialysis, ultrafiltration, precipitation, etc. Therefore, one optional step may comprise a step of: removing bioactive antifolate agent in aqueous solution outside of the liposomes after said extruding step.

Another example embodiment relates to a targeted liposomal composition that selectively targets folate receptors comprising: a liposome including an interior space, a bioactive agent disposed within said interior space, a steric stabilizer molecule attached to an exterior of the liposome, and a targeting moiety comprising a protein with specific affinity for at least one folate receptor, said targeting moiety attached to at least one of the steric stabilizer and the exterior of the liposome.

The components of this example embodiment may be the same as described for other embodiments of this disclosure. For example, the bioactive agent, the steric stabilizer which may be PEG, are as described in other parts of this disclosure.

In example embodiment, the bioactive agent of the example embodiment may be ellipticine; paclitaxel or any other bioactive agents listed in this disclosure. Agents related to ellipticine or paclitaxel are also envisioned. These include, at least, taxanes such as docetaxel and cabazitaxel.

The example embodiments further contemplate in vitro applications of the compositions and methods. In vitro use may be, for example, in the use such as cell culturing and tissue engineering where selective treatment of a subpopulation of cells are desired. For example, during the culture of stem cells from a normal patient or a patient suffering from cancer, the cells can be treated with a sample composition or sample liposome as discussed to address cancerous subpopulations of cells. The cancerous subpopulation may arise because the donor originally has cancer or because the cells spontaneously transform during in vitro procedures.

According to example embodiments, the liposomes and liposome compositions can be provided in a kit comprising a container with the liposomes, and optionally, a container with the entity and an instruction, e.g., procedures or information related to using the liposome composition in one or more applications. Such instruction can be provided via any medium, e.g., hard paper copy, electronic medium, or access to a database or website containing the instruction.

### EXAMPLES

The following examples are intended to illustrate but not to limit the invention in any manner, shape, or form, either explicitly or implicitly. While they are typical of those that might be used, other procedures, methodologies, or techniques known to those skilled in the art may alternatively be used.

Using the procedures of this disclosure including the procedures in the Example section, example compositions and example liposomes such as the liposomal antifolate composition are constructed. The example compositions comprise example liposomes. Both example composition and example liposome are used in the experiments described in the examples section and throughout this disclosure are specific embodiments of the disclosure and are not meant to define the full scope of the disclosure.

### Example 1: Production of folate receptor alpha targeted liposomes containing Pemetrexed and a Hapten

### Production of Pemetrexed Liposomes

Pemetrexed disodium heptahydrate (ALIMTA) is highly water soluble with a solubility of 100 mg/ml at neutral pH. Pemetrexed is encapsulated in liposomes by the following procedure. First, the lipid components of the liposome membrane are weighed out and combined as a concentrated solution in ethanol at a temperature of around 65°C. In this example, the lipids used are hydrogenated soy phosphitidyl choline, cholesterol, DSPE-PEG-2000 (1,2-distearoyl-*sn*-glycero-3-phosphoethanolamine-N-[methoxy(polyethylene glycol)-2000]), PEG-DSPE-malemide and PEG-DSPE-FITC. The molar ratio of HSPC: Cholesterol: PEG-DSPE is approximately 55:40:5. Next, Pemetrexed is dissolved in an aqueous buffer at a concentration of 100 mg/ml. The drug solution is heated to 65°C. The ethanolic lipid solution is injected into the Pemetrexed solution using a small bore needle. During this step the drug solution is well stirred using a magnetic stirrer. The mixing is performed at an elevated temperature (63°C -72°C) to ensure that the lipids are in the liquid crystalline state (as opposed to the gel state that they attain at temperatures below the lipid transition temperature Tm = 51°C -54°C). As a result, the lipids are hydrated and form multiple bilayer (multilamellar) vesicles (MLV) containing pemetrexed in the aqueous core.

### Downsizing of MLV's Using Filter Extrusion

The MLVs are fragmented into unilamellar (single bilayer) vesicles of the desired size by high-pressure extrusion using three passes through stacked (track-etched polycarbonate) membranes. The membranes used during the first pass have a pore size of 200nm. The membranes used during the second pass have a pore size of 100nm followed by 80 nm pore size membranes as the final pass. During extrusion the temperature is maintained above the Tm to ensure plasticity of the lipid membranes. As a result of the extrusion, large and heterogeneous in size and lamellarity MLVs turn into small, homogenous (80-100 nm) unilamellar vesicles (ULV) that sequester the drug in their interior. A Malvern Zetasizer Nano ZS instrument (Southborough, MA) with back scattering detector (90°) was used for measuring the hydrodynamic size (diameter) at 25°C in a quartz micro cuvette. The samples were diluted 50-fold in formulation matrix before analysis.

Our results show that liposomes down sized using filter extrusion had an average particle size of 85 nM with a PDI of 0.007 and a zeta potential of -43.7. As an alternative to filter extrusion, high pressure microfluidization can also be used to down size liposomes. We have been able to produce liposomes having a size from 40 nm and up, such as between 30-150 nm (data not shown) or even smaller than 30 nm, and particularly between 40 nm and 120 nm using methods such as high pressure filter extrusion or microfluidization alone or in combination.

### Tangential Flow Filtration (TFF) and Drug formulation

After the ULV's containing Pemetrexed have been produced, the extra-liposomal Pemetrexed is removed using dialysis or tangential flow diafiltration against a suitable buffer. Although any buffer solution can be used, in this example the buffer used was 5 mM Sodium Citrate, 60mM Sodium Chloride, pH 6.1. Upon completion of Dialysis, filter sterilize using 0.22 micron filter.

### Thiolation of Anti-Folate receptor alpha antibody

In order to conjugate the antibody to the PEG-DSPE-malemide moieties on the liposome, the antibody needs to be thiolated. In this example, thiolation of the antibody is achieved using Traut's reagent (Themo Fisher Scientific). The antibody is added to freshly prepared 14 mM Trauts reagent and 5 mM EDTA in phosphate buffered saline at a pH of 8.1. After incubation with gentle stirring for 60 minutes the thiolated antibody is separated from excess Trauts reagent by dialysis against 200 volumes of 25 mM HEPES pH 7.0, 60 mM NaCl for a minimum of 4 hours.

### Conjugation of Thiolated Antibody to the Pemetrexed Liposomes

The amount of thiolated antibody to be used is calculated based on the desired number of antibodies per liposomes. A 2 fold excess of of each preparation of thiolated antibody is added to diafiltered sterile liposomes. The reaction vessel is overlaid with Nitrogen gas and incubated overnight with slow stirring at room temperature of 4°C. The conjugation reaction is stopped by blocking unreacted maleimide groups by adding a stock aqueous 100mM L-Cysteine-HCl solution to a final concentration of 15 mM in the reaction mixture. Free thiolated antibody is then separated from the antibody conjugated liposomes by using size exclusion chromatography.

### Example 2: Cell lines used for Experiments

Cells lines used in the experiments are commercially available from sources such as the ATCC (American Type Culture Collection of Manassas, Virginia, U.S.A). The cell lines, their ATCC accession numbers and growth conditions are listed below.

Calu-3 (ATCC HTB-55); EMEM (Cat. # 30-2003); 10% HI FBS; 1% Pen/Strep; 1% L-Glutamine.

KB; EMEM (Cat. # 30-2003); 10% HI FBS; 1% Pen/Strep; 1% L-Glutamine.

CCD841 (ATCC CRL-1790); EMEM (Cat. # 30-2003); 10% HI FBS; 1% Pen/Strep; 1% L-Glutamine.

Hs578Bst (ATCC HTB-125); Hybri-Care Medium pH 7.0 (Cat.# 46-X); 30 ng/ml mouse EGF; 10% HI FBS; 1% Pen/Strep; 1% L-Glutamine.

NCI-H2087 (ATCC CRL-5922); RPMI-1640 (Cat. # 30-2001); 5% HI FBS; 1% Pen/Strep; 1% L-Glutamine.

NCI-H2452 (ATCC CRL-5946); RPMI-1640 (Cat. # 30-2001); 10% HI FBS; 1% Pen/Strep; 1% L-Glutamine.

OVCAR-3 (ATCC HTB-161); RPMI-1640 (Cat. # 30-2001); 20% HI FBS; 1% Pen/Strep; 1% L-Glutamine.

SKBR3; McCoy 5A Medium; 10% HI FBS; 1% Pen/Strep; 1% L-Glutamine.

SL0003 (ATCC PTA-6231); F-12K Medium; 10% HI FBS; 1% Pen/Strep; 1% L-Glutamine.

A549 (ATCC CCL-185); F-12K Medium; 10% HI FBS; 1% Pen/Strep; 1% L-Glutamine.

### Example 3 Determining binding specificity of one sample construct

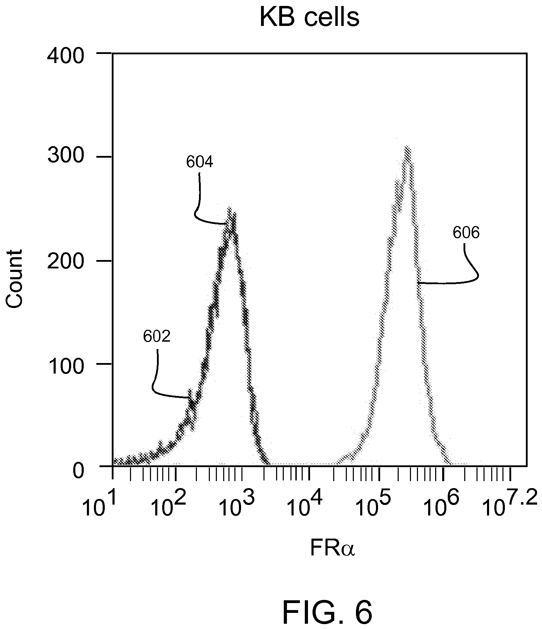

The level of folate receptor alpha on the cell surface was measured by flow cytometry with a monoclonal antibody conjugated with a fluorochrome. A shift to the right after binding of an antibody (see, for example, Figure 6, line 606) compared to the line before antibody treatment (see, for example, Figure 6, line 602 and 604) indicates the detection of receptor by flow cytometry. The more the histogram (e.g., Figure 6, line 606) shifts to the right relative to the untreated cells (see, for example, Figure 6, line 602 and 604) the higher the levels of receptors are on the cell surface. The plots demonstrate high levels of folate receptor alpha on cancer cells, but almost undetectable levels on normal cells.

The example liposome which is part of the example liposomal composition constructed, binds to the cell surface to cells that are folate receptor alpha positive, but not cells which are folate receptor alpha negative. The example liposome contains antibody to folate receptor alpha. When the example liposome is incubated for a short period (30 minutes) with folate receptor alpha+ cells, you can detect the example liposome on the cell surface by measuring the level of FITC integrated in the liposome by flow cytometry. A shift of the peak of the histogram indicates that the example liposome is detected on the cell surface. The more the peak shifts to the right, the more example liposome is detected on the cells.

In this experiment we determined the binding of example liposome to cells to access their affinity and specificity. Briefly the example liposome which comprises a detectable label, were coincubated with cells and the cells were analyzed by flow cytometry. The following data shows that the example liposome binds to folate receptor alpha positive cancer cells, but not folate receptor alpha negative, normal cells.

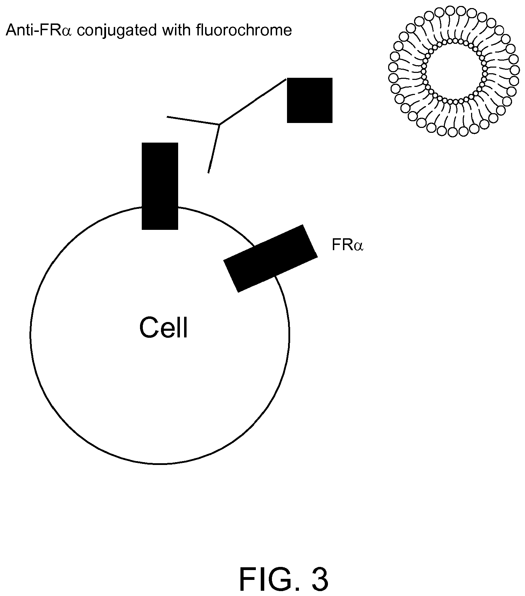

Figure 3 is a schematic depicting the measurement of folate receptor alpha on the cell surface.

Figure 6 is a representative histograms of KB cancer cell lines expressing high surface levels of folate receptor alpha as measured by flow cytometry. In Figure 6, label 602 = no antibody; label 604 = isotype control; label 606 = anti-folate receptor alpha APC.

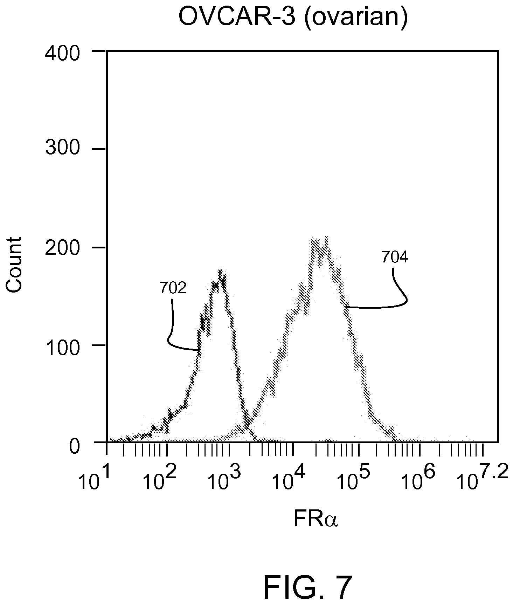

Figure 7 is a representative histograms of OVCAR-3 cancer cell line expressing high surface levels of folate receptor alpha as measured by flow cytometry. In Figure 7, label 702 = no antibody; label 704 = anti-folate receptor alpha APC.

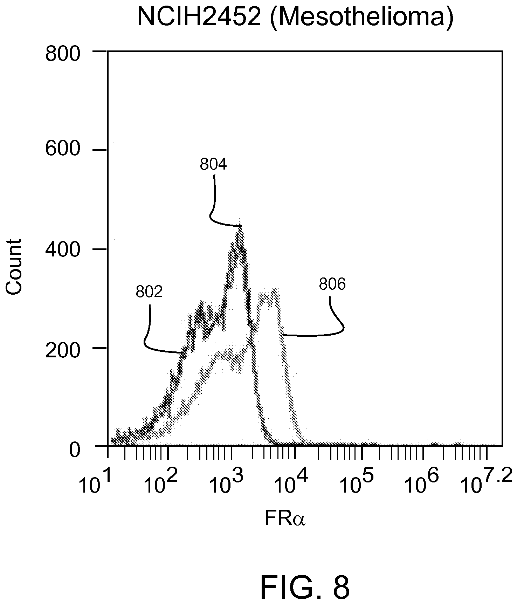

Figure 8 is a representative histograms of NCIH2452 cancer cell line expressing high surface levels of folate receptor alpha as measured by flow cytometry. In Figure 8, label 802 = no antibody; label 804 = isotype control; label 806 = anti-folate receptor alpha APC.

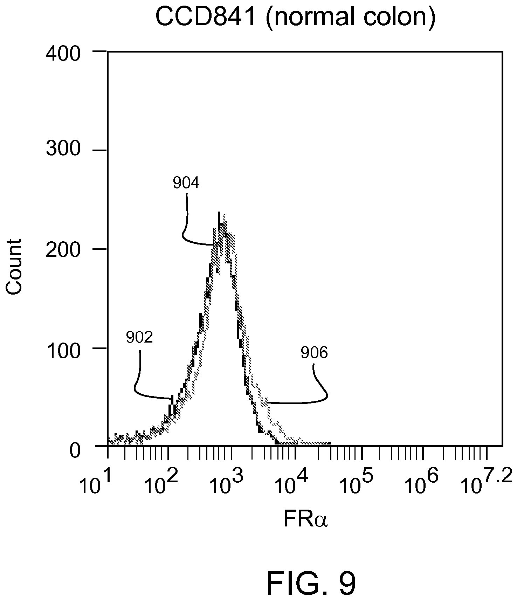

Figure 9 is a representative histogram of normal cell line derived from colon epithelia expressing low surface levels of folate receptor alpha. In Figure 9, label 902 = no antibody; label 904 = isotype control; 906 = anti-folate receptor alpha APC.

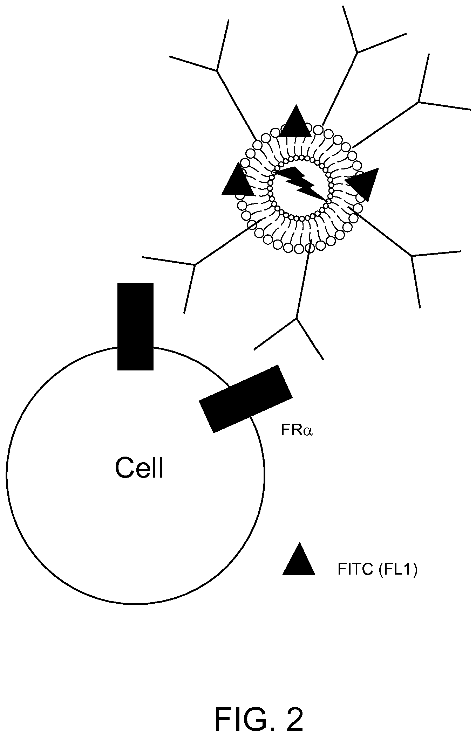

Figure 2 is a schematic depicting an example liposome binding to the cell surface.

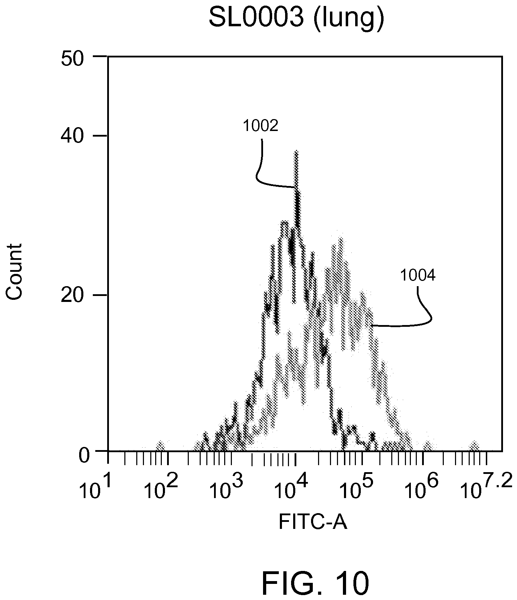

Figure 10 is a representative histograms of SL0003 (lung) cell line. The lung cancer cell line binds high levels of the example liposome. Label 1002 = untreated cells. Label 1004 = the example liposome treated cells.

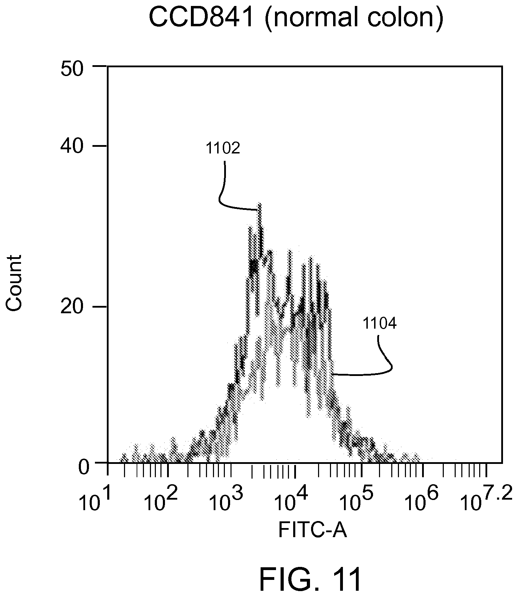

Figure 11 is a representative histograms of CCD841 (normal colon) cell line. folate receptor alpha-negative cell line bind little example liposome. Label 1102 = untreated cells. Label 1104 = example liposome treated cells.

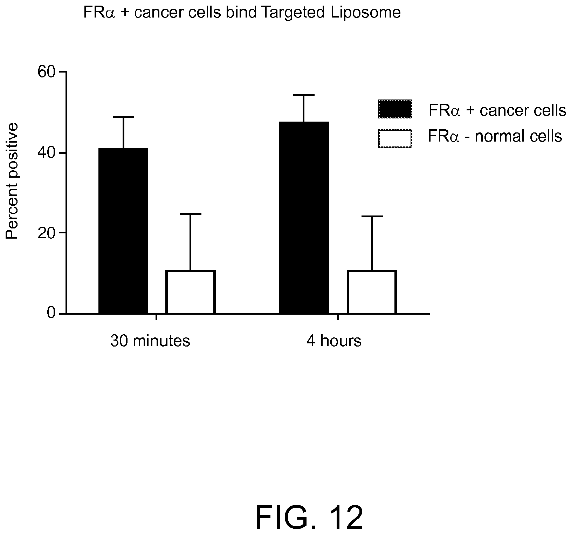

Figure 12 shows composite data derived from lung (SL0003) and ovarian (OVCAR-3) cells demonstrating high levels of example liposome binding on the cell surface compared to normal cells derived from colon (CCD841) and breast (Hs578), P<0.05. Shown are surface levels of example liposome detected at 30 minutes or 4 hours of incubation at 37°C.

In these experiments, the assays were performed as follows:
Cell were collected and washed in 0.2% Bovine serum albumin in PBS (FACS buffer.) Cell were resuspended in 100 µ1 volume in FACS buffer. 5 µl of anti-folate receptor alpha monoclonal conjugated with APC was added (cat# FAB5646A; R&D Systems). The cells were incubated for 30 min in the dark at 4°C. 100 µl of FACS buffer was added to wash the cells and then the cells were evaluated by flow cytometry (FL4). For measuring the example lyposome on cell surface. Cell were collected, counted, and washed in 0.2% Bovine serum albumin in PBS (FACS buffer.). 20,000 cells were resuspended100 µl volume in FACS buffer. 2 µl of the example liposome was added to the cells. The cells were incubated at 37°C for 30 minutes, washed with FACS buffer, and evaluated by flow cytometry.

### Example 4 RhodoRed Experiment on Sample Compositions and Sample Liposomes

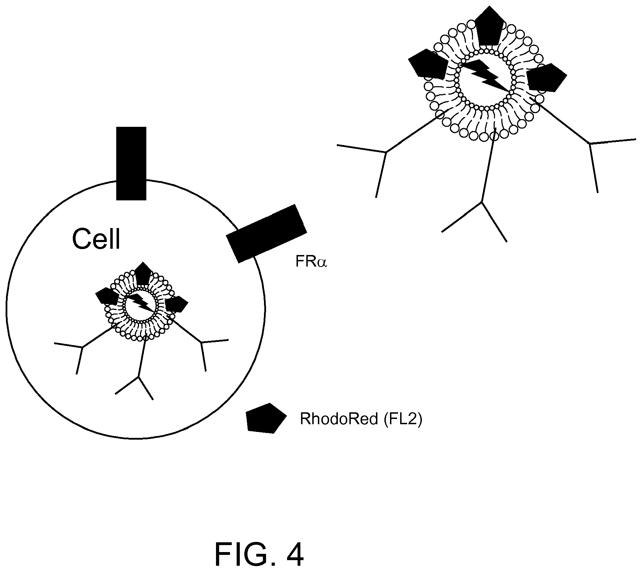

Figure 4 Schematic depicting sample liposome in the cell. Sample liposome was labeled with pH-RhodoRed, which fluoresces in the presence of reduced pH, such as in the endolysosome of the cell. Internalization is seen as a shift to the right of the peak relative to untreated cells.

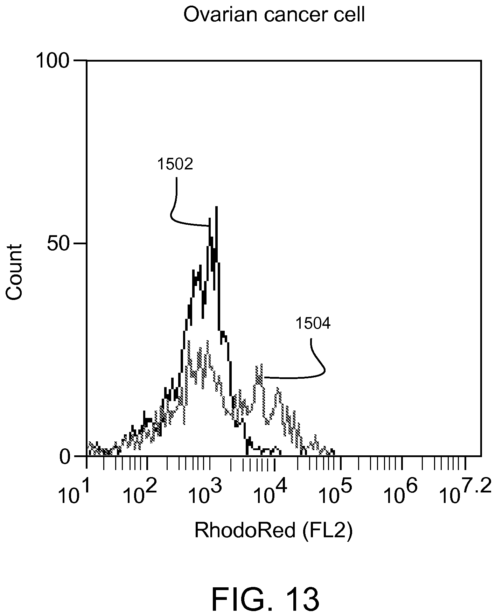

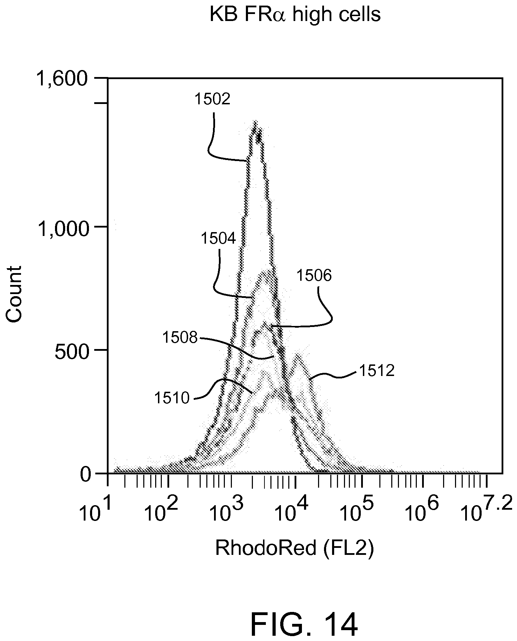

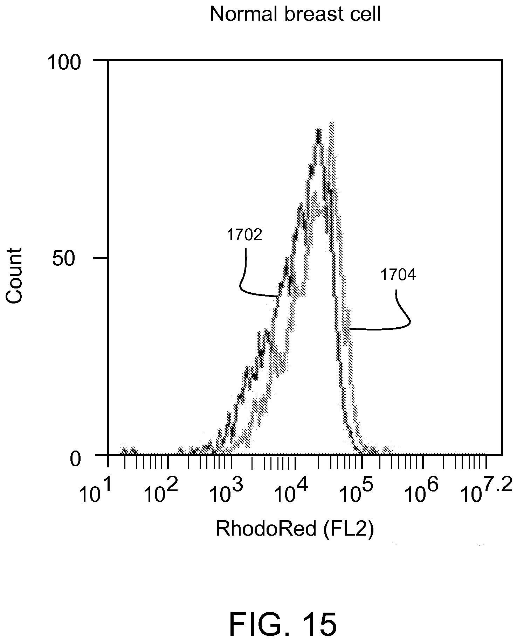

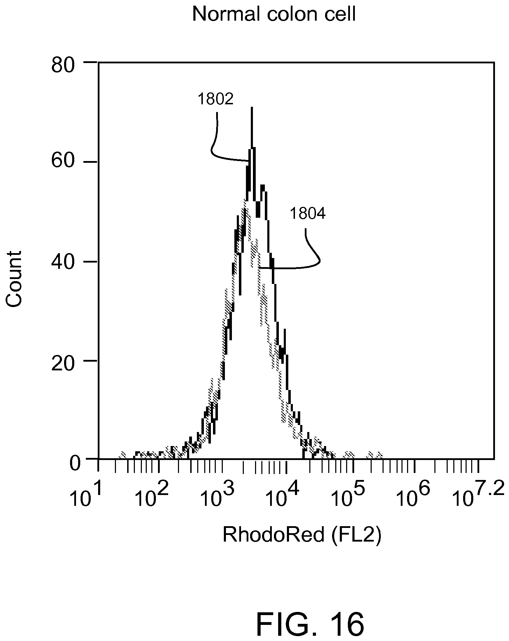

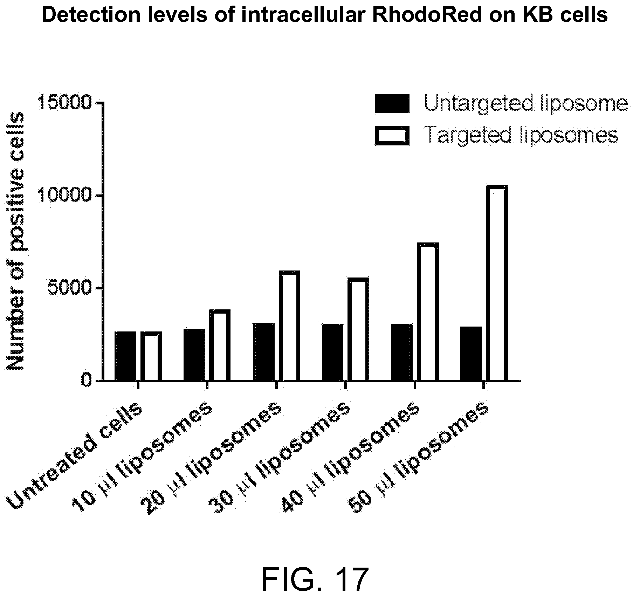

Figure 13 shows that RhodoRed-labeled cells of the example liposome is internalized by ovarian cancer cells. This is evident because peak 1504 (cells treated with the example composition/example liposome) is shifted to the right of the untreated peak 1502. Similarily, in Figure 14, we see that relative to the untreated peak 1502, the treated peak with increasing amounts of example liposome, begin to shift right as seen in peaks 1504, 1506, 1508, 1510 and 1512 referring to 10 µl, 20 µl, 30 µl, 40 µl, and 50 µl respectively (see Figure 17). The same data is plotted in a bar chart in Figure 17. In Figure 17 a control pH-RhodoRed-labeled liposome lacking anti-folate receptor a (FOLR1) was assessed for comparison. Liposomes lacking anti-FOLRlwere not internalized by KB cells. In contrast, pH-RhodoRed labeled sample liposome was internalized by KB cells. This data in Figure 17, as shown, is the result of 18 hour incubation at 37°C, dose response, also quantified in Figure 14. Folate Receptor alpha negative normal cell lines (breast cell; left panel and colon; right panel) did not internalize pH-RhodoRed labeled sample liposome. Figures 15 shows that internalization is minimal in normal breast cells. Peak 1704 is only slightly shifted relative to peak 1702. Figure 16 shows that internalization is minimal in normal colon cells as peak 1802 and 1804 were not substantially shifted.

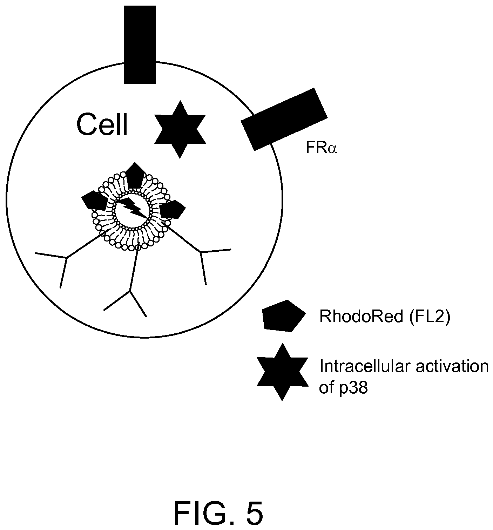

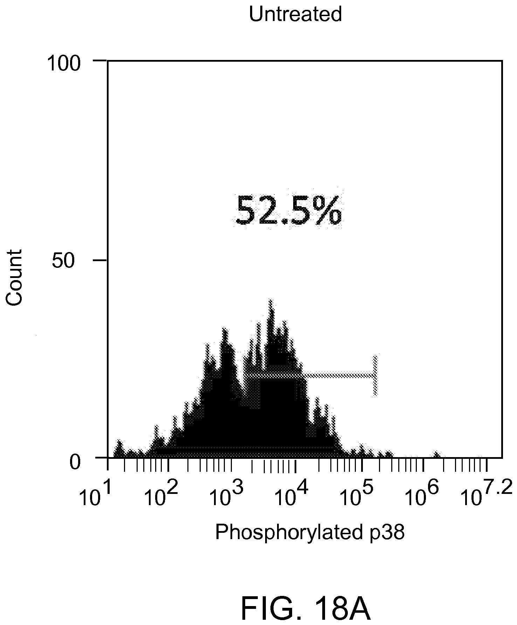

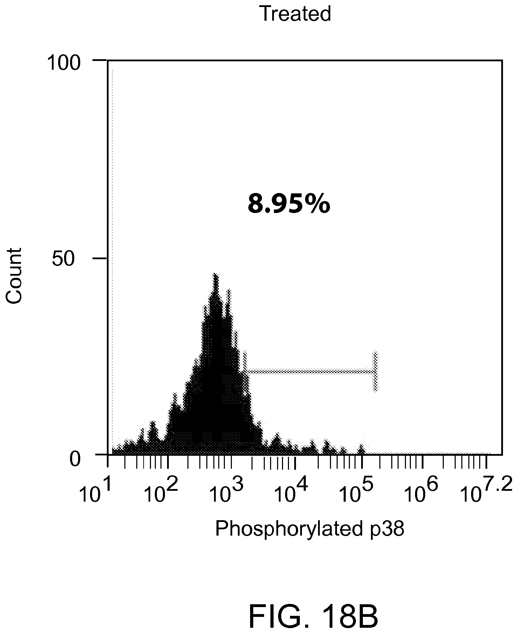

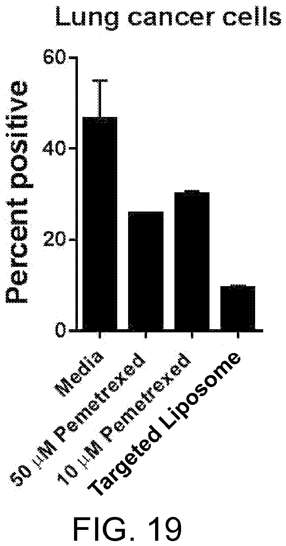

To further evaluate the internalization, SL0003 lung cancer cells were assessed for MAP kinase activation levels by PhosFlow. Schematic depicting sample liposome inside the cell activating kinases is shown in Figure 5. Figure 18A and Figure 18B shows the effect of pemetrexed on the reduction of basal levels of phosphorylation of p38 at 30 minutes post-treatment. Figure 18A shows untreated cells with 52.5% phosphyrylated p38. When the cells were treated with the example composition comprising the example liposome in Figure 18B, this percentage is reduced to 8.95%. Figure 19 shows the quantification of phosphorylated levels of p38 in cancer cells and normal cells at 30 minutes post-treatment. The sample composition and sample liposome, labeled as "Targeted Liposome," affects p38 activation in SL0003 lung cancer cells.

We interpret the data as follows: Sample liposome enters cells that are FOLR1 positive. sample liposomes were labeled with a dye (RhodoRed) that can only be detected by flow cyometry if it enters the cell. Various cells were incubated with differing amounts of RhodoRed-labeled sample liposome and the level of fluorescence was measured by flow cytometry (FL2.) RhodoRed labeled sample liposomes enters cancer cells that express FOLR1, but not normal cells that are FOLR1 negative. Shown are ovarian cancer cells with the peak shifting to right indicating the drug has entered the cell (Figure 13). The same experiment was done with FOLR1 high KB cells with titrated amounts of RhodoRed-labeled sample liposome (Figure 14). These data are quantified in Figure 17. Sample liposomes entered KB cells when treated with high concentrations but the control liposomes that lacked anti-FOLRl antibody were not able to enter the cell.

A second measure of sample liposome entering the cell is intracellular activation pathways. Cells respond to ligands binding to their receptors by activating kinases, in this case p38. The activated kinases can be measured by flow cytometry. The cells are incubated with sample liposome for 30 minutes and then lysed with a mild detergent. The activated p38 kinase is detected with an antibody by flow cytometry. A shift under the red line gate indicates a higher level of phosphorylated p38 (See Figure 18A and 18BG). Cancer cells may have a higher basal level of activated kinases. In this case, pemetrexed reduces p38 activation similarly to sample liposome demonstrating that the pemetrexed inside sample liposome is active (Figure 19).

Experimental conditions are as follows:
For Figures 4, 13, 14, 15, 16 and 17: Measuring uptake of RhodoRed -labeled sample liposome. Cells (OvCAR-3, KB, normal colon and normal breast) were plated cell at 7,000 cell/well the night before the experiment. The next day, cells were treated with the following 1) No drug 2) RhodoRed-labeled anti-FOLRl monoclonal antibdy (MABFRAH H/L) Ab - (1ul); 3) sample liposome (Liposome FOLR1 Ab conjugated - (5ul); 4) Non targeted pH Rhodored liposome - (5ul)/ Liposomes with no anti-FOLRl. The cells were incubated at 37°C for 18 hours and 24 hours. 100 µl of ice cold FACS buffer was added and the cells were evaluated by flow cytometry (FL2).

In addition, for Figure 17, measuring p38 phosphorylation (PhosFlow) SL0003, normal colon cells, and normal breast cells were seeded at 10,000 cells/well. Cells were treated with: Fresh Pemetrexed (50 µM, 10 µM), LEAF-001 (Liposome 070715 MPF; 5traut/50 Mab, 15traut/50Mab, 45traut/50Mab) (13.33X dilution) , anti-folate receptor alpha (MABFRA H/L 1.01 mg/ml) (13.33X dilution) to determine the effect of the antibody in sample liposome, or no treatment. The cells were gently mixed and quickly placed in incubator. At each time point (30 minutes- 4 hours), the plates were removed from the incubator and immediately fixed with formaldehyde for a final concentration of 2%. The plates were incubated for 5 minutes at room temperature. 25 µl of media was removed. 25 µl of FACS buffer was added. 100 µl of cell lysis buffer (FACS buffer, 0.2% triton X-100, 0.3% formaldehyde) were added. Cells were collected into 1.5 ml centrifuge tubes and vortexed for 3 minutes to lyse the cells. The PE-conjugated anti-P38 (BD Pharmingen) antibody or PE-conjugated isotype control was added and the cells were incubated for 30 minutes in the dark at 4oC. 200 µl FACS buffer was added to wash. The tubes were spun and the supernatant was carefully removed. The cells were read on the flow cytometer (in FL2).

### Example 5 MTS Assay

The MTS (3-(4,5-dimethylthiazol-2-yl)-5-(3-carboxymethoxyphenyl)-2-(4-sulfophenyl)-2H-tetrazolium) assay is a well-known colorimetric assay for assessing cell metabolic activity. The cell lines were used for the assays and their growth conditions are as follows:
(a) Calu-3: EMEM, 10% HI FBS, 1% Pen/Strep, 1X L-glutamine;
(b) KB: EMEM, 10% HI FBS, 1% Pen/Strep, 1X L-glutamine;
(c) NCI-H2087: RPMI, 5% HI FBS, 1% Pen/Strep, 1X L-glutamine;
(d) NCI-H2452: RPMI, 10% HI FBS, 1% Pen/Strep, 1X L-glutamine;
(e) SKBR3: McCoy's, 10% HI FBS, 1% Pen/Strep, 1X L-glutamine;
(f) CHO: FreeStyle CHO, 5% HI FBS, 1%Pen/Strep, 1X L-glutamine;
(g) A549: F-12K, 10% HI FBS, 1%Pen/Strep, 1X L-glutamine; and
(h) SL0003: F-12K, 10% HI FBS, 1%Pen/Strep, 1X L-glutamine.

The night before, the cells are seeded according to the amount of cells required for each cell line in 96 well tissue culture plate. Final volume in each well is 100 µL (see Table of cell lines for reference; all cell lines obtained from ATCC.). Cell line used and assay conditions are as follows:
(1) Calu-3: 10000 cells per well. Stock is 3.1x10⁵/ml: diluted 3.55mL of cell to 7.45mL of completed media. Transfer 100 µl to each well (µl refers to microliter).
(2) KB: 3000 cells per well. Stock is 2.0×10⁵/ml: diluted 1.65mL of cell to 9.35mL of completed media. Transfer 100 µl to each well.
(3) NCI-H2087: 3000 cells per well. Stock is 3.7×10⁵/ml: diluted 892 µl of cell to 10.1mL of completed media. Transfer 100 µl to each well.
(4) NCI-H2452: 5000 cells per well. Stock is 5.0×10⁴/ml: no need for dilution
(5) SKBR3: 4000 cells per well. Stock is 5.5×10⁵/ml: diluted 800 µl cell to 10.2mL of completed media. Transfer 100 µl to each well.
(6) CHO: 3000 cells per well. Stock is 3.6×10⁵/ml: diluted 1mL cell to 11 mL of completed media. Transfer 100 µl to each well.
(7) A549: 3000 cells per well. Stock is 2.3×10⁵/ml: diluted 1.43mL cell to 9.57mL of completed media. Transfer 100 µl to each well.
(8) SL0003: 3000 cells per well. Stock is 2.3×10⁵/ml: diluted 1.43mL cell to 9.57mL of completed media. Transfer 100 µl to each well.

The seeded cells are incubated at 37°C and 5% CO2 overnight. The next day, the drugs were prepared in the cell-specific cell culture media and titrated 2-fold diluted concentrations added to the cells. The preparations are as follows:
Pemetrexed heptahydrate (5 mM stock). Top dilution 10uM: Add 2 µl of stock to 998 µl of completed media.

Example liposome/Liposome FOLR-1 Ab (0.4mg/ml = 666.67 µµM). Top dilution 10 µM: Add 9 µl of stock to 591 µl of completed media.

Liposome Lot 0707F (stock is 2mM). Top dilution 10 µM: Add 3 µl of stock to 597 µl of completed media.

On day 4, the effect on cellular proliferation was measured with MTS assay. 10 µl of reagent (Celltiter 96® Aqueous One Solution) were added to each well. This is a colormetric assay that turns a deep purple when there is extensive cellular proliferation. The plates were incubated for 2 hours at 37oC and the absorbance was measured at 490nm. Percent inhibition of cell growth was calculated using the untreated cell absorbance values set at 100% for each cell line.

### Example 6 Sample Liposome Effect on Cellular Proliferation

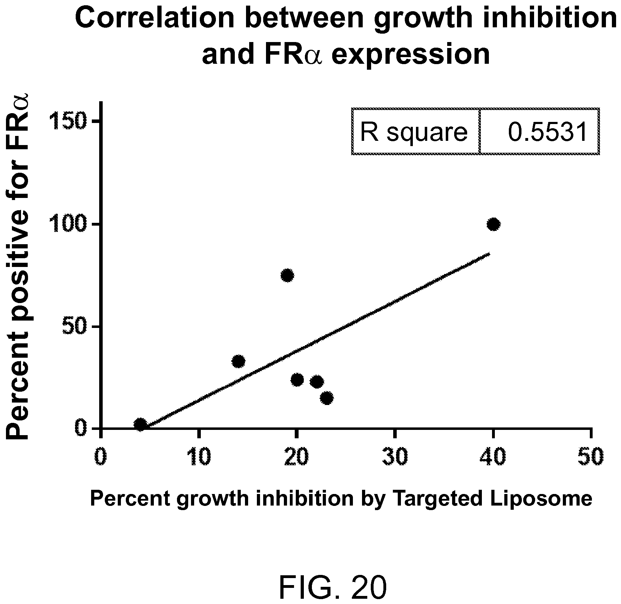

Figure 20 shows folate receptor alpha surface levels on cancer cells correlate with susceptibility to sample liposome growth inhibition. Shown are the levels of inhibition in A; p=0.05. To further assess the effect of sample liposome on cell cycle, SL0003 (lung cancer) cells were treated with pemetrexed or sample liposome for 4 days.

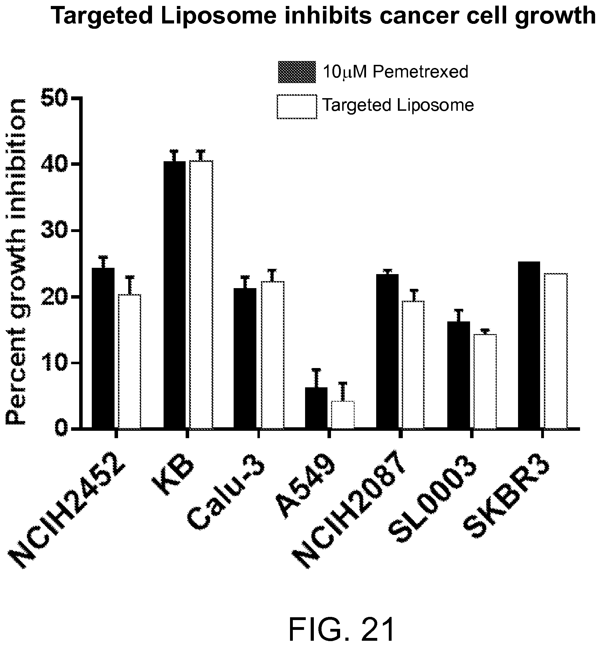

Figure 21 is a chart showing cell lines derived from patient with lung or breast cancer were treated with titrated concentrations of pemetrexed or the example liposome. Cells were incubated for 90 hours at 37°C and cellular viability and number were assessed by MTS. Shown are results from 10 mM pemetrexed compared to sample liposome with estimated 10 mM pemetrexed. Sample liposome demonstrates a similar efficacy as pemetrexed.

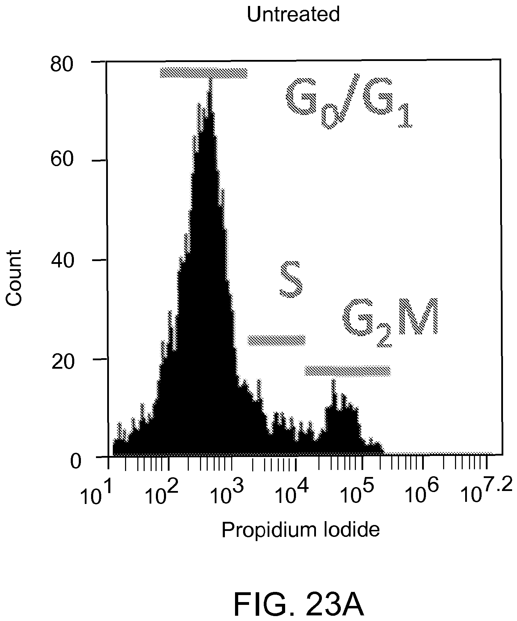

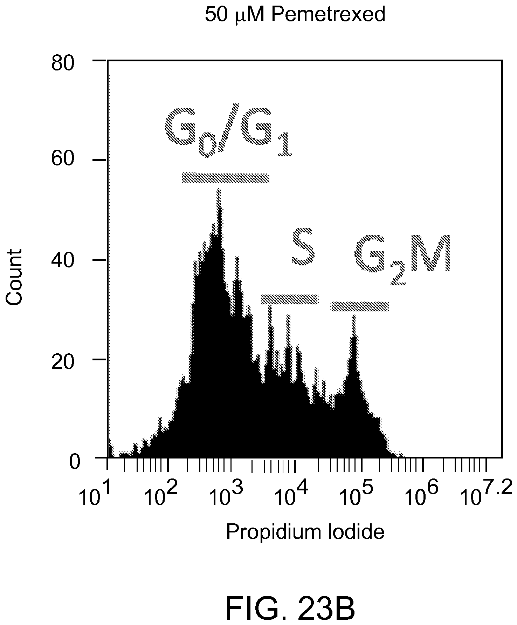

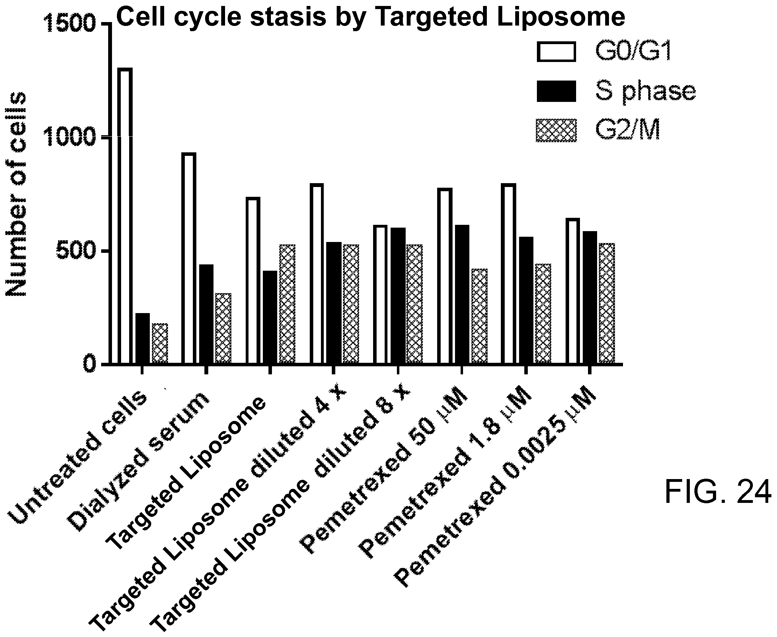

Cells were lysed and the DNA labeled with propidium idodine to quantify cell cycle (Figure 23A) Pemetrexed treatment induces cells to accumulate in S phase (Figure 23B) Composite data demonstrating that sample liposome induces the same effect on cell cycle as pemetrexed with an accumulation of cells in S phase is shown in Figure 24.

This data shows that pemetrexed is an effective chemotherapy by stopping cancer cells from dividing. We tested whether the pemetrexed contained within sample liposome was effective in inhibiting cells from dividing. Several cancer cell lines were treated with either 10 mM pemetrexed or sample liposome with an estimated matched concentration of pemetrexed for 4 days. The cells were then assessed for numbers of cells that divided. The data show that sample liposome and pemetrexed have similar effects on each of the cell lines.

The FOLR1 expression on the cell surface (see Figure 20) correlates with susceptibility to sample liposome. We used a second measure of the effects of pemetrexed on the ability of cells to divide. Cells treated with pemetrexed cannot produce new DNA and become trapped in the S phase of the cell cycle. Sample liposome has the same effect as pemetrexed.

### Example 7 Evaluation of the effect of sample liposome on cell cycle

SL0003 (lung cancer) cells were prepared as described in the Example describing MTS assays (Example 5).

For this assay, the cells were cultured for 4 or 5 days (shown is day 5.). More specifically, SC0003 cells (lung adenocarcinoma) were seeded in 96 well plates and treated the next day with titrated concentrations of LEAF-001 or pemetrexed. At day 5, cells were lysed with FACS buffer, 0.2% triton X-100, 0.3% formaldehyde. The DNA was stained with Propidium Idodine for 30 minutes was labeled with Propidium Iodide to evaluate cell cycle

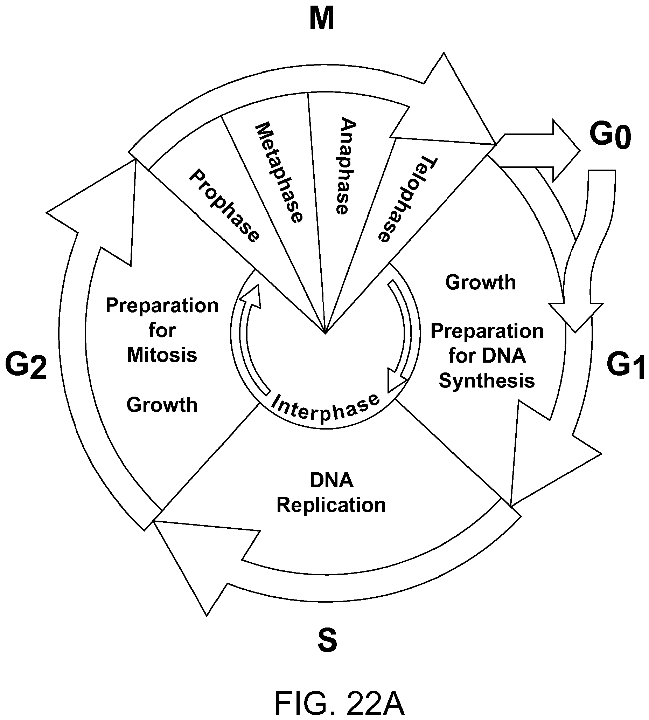

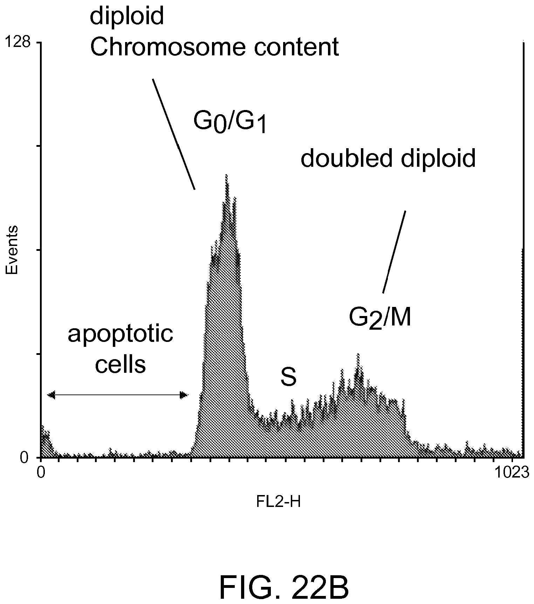

The cells were washed and evaluated by flow cytometry (FL2). Figure 22A depicts a schematic showing the cell cycle. The experimental results are shown in Figure 22B. By inhibiting the formation of precursor purine and pyrimidine nucleotides, pemetrexed prevents the formation of DNA and RNA, which are required for the growth and survival of both normal cells and cancer cells.

### Example 8 Sample liposome reduces the toxicity of pemetrexed on bone marrow-derived neutrophils.

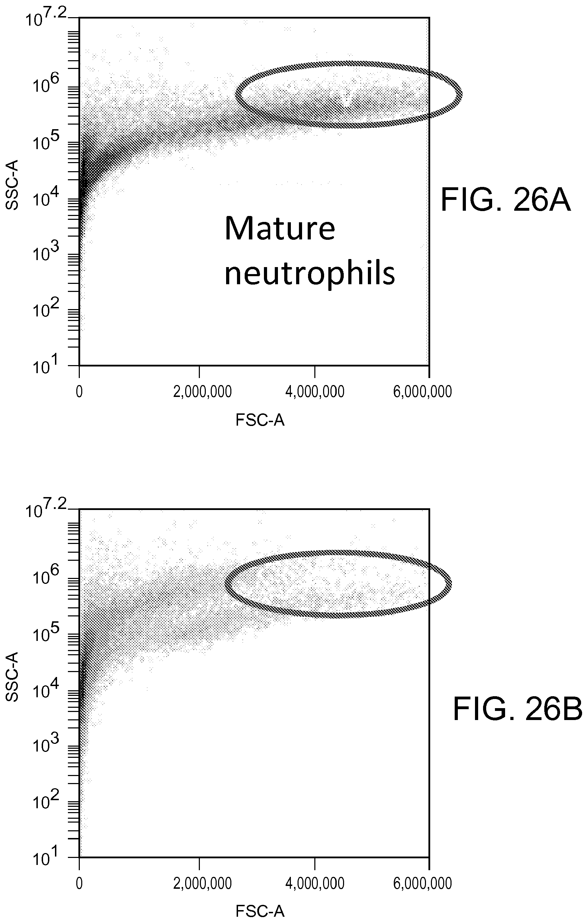

CD34+ cells were induced to differentiate into neutrophils with IL-3, stem cell factor, and G-CSF. By day 2, there is a dramatic increase in mature neutrophils depicted in the oval (Figure 26A). In the presence of pemetrexed (2-50 mM), neutrophil differentiation is inhibited Figure 26B; n=4 donors).

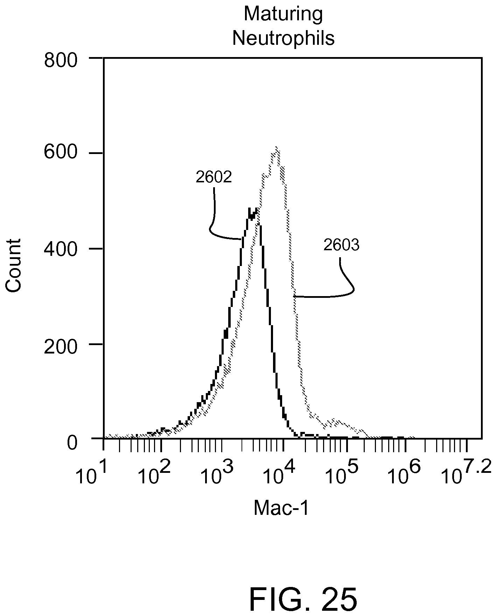

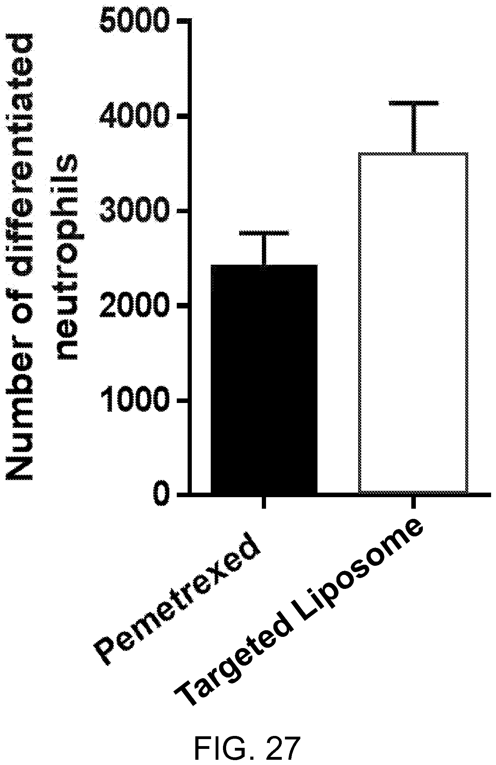

Mac-1 expression on neutrophils in drawn circles in Figure 25A and Figure 25B is shown in Figure 25. As can be seen in Figure 27, sample liposome (at 10 mM pemetrexed) reduces the toxicity of pemetrexed on neutrophil differentiation (n=3 donors.) Cells were treated with a calculated estimation of sample liposome at 10 mM pemetrexed for two days. Numbers of differentiated neutrophils were assessed by flow cytometry as shown in Figure 26A and 26B. The circle denotes maturing neutrophils expressing Mac-1 and CD15.

One of the side effects from pemetrexed treatment is the reduction of neutrophils in the bloodstream. This is the result of CD34+ stem cells not differentiating, or developing, into mature neutrophils in the bone marrow. We measured the effect of sample liposome on neutrophil differentiation compared to the same dose of pemetrexed (10mM.) Stem cells from 4 donors were purchased and treated with a panel of growth factors to induce neutrophil differentiation. CD34+ cells that were also treated with pemetrexed failed to develop into mature neutrophils.

The level of a molecule called Mac-1 is elevated on more mature neutrophils. This molecule is elevated on cells in the circles drawn on the plots. A shift to the red indicates increased levels on the cells.

In contrast, sample liposome was able to reduce this toxicity by allowing more cells to develop into neutrophils. See, e.g., Figure 27.

Experiments were performed as follows: CD34+ stem cells were obtained from ATCC. CD34+ cells were thawed at 37°C for 1 minute. While on ice, the cells were transferred to cold stem cell medium ("StemSpan SFEM" - Stem Cell Tech. cat.# 9650), 10% heat activated fetal bovine serum (HI FBS.) Each vial contained approximated 5x105 cell/ml. The cells were placed in 96 well tissue culture plates ~ 35,000 cell/well.

The neutrophils GROWTH media contained 100 ng/ml of stem cell factor human (SCF-Sigma H8416, lot# MKBT8036V), 20 ng/ml of granulocyte colony- stimulation factor, human (G-CSF- Sigma H5541, lot # SLBC9602V), 10 ng/ml of IL3 recombinant human (Sigma SRP3090, lot #1008AFC13) in StemSpam media as above.

The cells were also treated as follows 1) StemSpam media alone with no growth cytokines; 2) StemSpam media + growth cytokines, 3) 50, 10 or 2 µM pemetrexed, 4) sample liposome (equivalent to 10 µM pemetrexed), or 5) anti-folate alpha Ab (1.01 mg/ml) - 5ug/ml

Cells were incubated for 1-5 days and assayed at each time point for mature neutrophils by flow cytometry with antibodies to CD15, Mac-1, and CD34. The cells shown in the circle on the plots are maturing neutrophils expressing Mac-1 and CD34.

### Example 9 Results and Discussion

Folate receptor alpha expression is restricted to specific organs beyond the fetal/embryonic stage in humans in noncancerous states. As shown in Figure 1A, in the setting of normal polarity, normal simple epithelium comprises a monolayer of individual cells that display a distinct apical- basal polarity. Cells are tightly packed and connected to each other by the apical junctional complexes, which separate apical and basolateral membrane domains (Figure 1A label 101). In normal tissue where polarity is preserved, folate receptor alpha is attached at the apical surface of cells situated away from, and out of direct contact with folates in the blood circulation (Figure 1A label 102). By contrast, disruption of cell polarity and tissue disorganization is a hallmark of advanced epithelial tumors. Figure 1B shows how cells in high-grade epithelial tumors display loss of apical-basal polarity and overall tissue disorganization, putting folate receptor alpha in direct contact with folates in the blood circulation (Figure 1B, label 103). In addition, tumor tissue cells in general express higher levels of folate receptor alpha than normal cells that happen to express this receptor. This differentiating feature of tumor tissue cells from normal epithelial cells is at the core of the design of the new chemical entity designed to rehabilitate antifolates as anticancer therapies while minimizing associated severe and sometime life-threatening toxicities. Such chemical entity delivers an antifolate agent in a manner that selectively targets specifically folate receptor alpha, not with folic acid but with a folate receptor alpha specific targeting moiety to bypass RFCs. This approach limits the exposure of the antifolate to tumor tissue cells only due to loss of cell polarity, because these tumor tissue cells overexpress folate receptor alpha during the time this receptor is in direct contact with blood circulation. This is not the case for limited normal tissues where folate receptor alpha is expressed, because the receptor is not in direct contact with circulating blood.

From this point on, folate receptor alpha can also be used interchangeably with folate receptor alpha that describes the gene encoding the folate receptor alpha protein. Both terms are used interchangeably to describe the folate receptor alpha protein. In addition, the new chemical entity will be referred, for purpose of illustration, the example liposome (also referred to as the targeted liposome). Methods for making the example compositions and example liposomes are disclosed throughout the specification and at least in Example 1. The discussion below refers to some experiments performed on a few example compositions and a few example liposomes. It is not meant to define all possible example compositions and all possible example liposomes.

Figure 2 illustrates the example liposome and how it binds to a cell that expresses folate receptor alpha. In addition to being a hapten, FTIC serves as an imaging agent that allows visualization of binding of the example liposome to the folate receptor alpha on the surface of a folate receptor alpha-expressing cell while Figure 3 illustrates the construct designed on one hand to document binding to the folate receptor alpha and, on the other hand, to quantify the number of folate alpha receptors exposed on the cell surface.

Figure 4 illustrates internalization of the example liposome into a folate receptor alpha expressing cell using RhodoRed. The exercise is to demonstrate that the example liposome is internalized independent of bioactive agent payload. Figure 5 illustrate further the effect of internalization of the example liposome on the cell proliferation using p38 protein kinase pathways as a read out of the cellular response to stress.

The next series of illustrations (Figures 6-11) describe the experiments and results showing first that the folate receptor alpha targeting antibody used binds preferentially folate receptor alpha. In these experiments, the level of folate receptor alpha on the cell surface was measured by flow cytometry with a monoclonal antibody conjugated with a fluorochrome. A shift to the right indicates the detection of receptor by flow cytometry. The more the histogram shifts to the right, the higher the levels of receptors are on the cell surface. The plots demonstrate high levels of folate receptor alpha on cancer cells (Figures 6-8), but almost undetectable levels on normal cells (Figure 9).

The example liposome can have an antibody targeting preferentially folate receptor alpha. When the example liposome is incubated for a short period (30 minutes) with folate receptor alpha positive cells, you can detect example liposome on the cell surface by measuring the level of FITC integrated in the example liposome by flow cytometry. A shift of the histogram line to the right indicates that the example liposome is detected on the cell surface. The more the histogram shifts to the right, the more the example liposome drug is detected on the cells. The experiments show that the example liposome binds to folate receptor alpha expressing lung cancer cells (Figure 10) but not to normal colon epithelial cells (Figure 11).

The example liposome binding experiments described above were repeated using multiple cancer cell lines overexpressing folate receptor alpha (lung-SL0003 and ovarian-OVCAR-3) and normal cells derived from colon (CCD841) and breast (Hs578) tissues. Figure 12 shows that the composite data derived from lung (SL0003) and ovarian (OVCAR-3) cancer cells, which have high levels of cell surface folate receptor alpha, demonstrate significantly higher levels of the example liposome binding on the cell surface compared to normal cells derived from colon (CCD841) and breast (Hs578) (with a p-value <0.05). Data shown in Figure 12 comprise surface levels of the example liposome detected at 30 minutes and at 4 hours of incubation at 37 degrees Celsius.

Another series of experiments was conducted to show that upon binding to folate receptor alpha expressing cells, the example liposome is further taken into the cells (internalized). This was assessed in two ways:
First, the example liposome liposomes were labeled with a dye (RhodoRed) that can only be detected by flow cytometry if it enters the cell (Figure 4). Various cells were incubated with differing amounts of RhodoRed-labeled example liposome and the level of fluorescence was measured by flow cytometry (FL2). Shown in Figure 13 are ovarian cancer cells with a shift to right indicating the example liposome has entered the cell. RhodoRed labeled example liposome enters cancer cells that express folate receptor alpha (Figure 13-14), but not normal cells that are folate receptor alpha negative (Figure 15-16).

The same experiment was specifically conducted in high folate receptor alpha expressing KB cells this time with titrated amounts of RhodoRed-labeled the example liposome. As shown in Figure 17, the example liposome entered KB cells when treated with high concentrations but the control liposomes that lacked anti-folate receptor alpha antibody were not able to enter the cell.

Taken together, these results from the RhodoRed experiments provide evidence that the technology used in the design construct of the example liposome is such that the example liposome as a delivery system, armed with a folate receptor targeting moiety other than folic acid or its analogues, enters cancer cells expression folate receptor alpha regardless of its liposome bioactive agent payload. Furthermore, the same experiments demonstrate preferential targeting of folate receptor alpha expressing cancer cells by the example liposome while limiting exposure of normal cells to the bioactive agent payload.

A second measure of the example liposome entering the cell was based on assessing intracellular activation pathways. Cells respond to stress from ligands binding to their receptors or internalization by activating p38 protein kinase pathways (Figure 20). The activated kinases can be measured by flow cytometry. The cells were incubated with the example liposome for 30 minutes and then lysed with a mild detergent. The activated p38 kinase was detected with an antibody by flow cytometry. Cancer cells may have a higher basal level of activated kinases (Figure 18A). A shift under the control line gate indicates a higher level of phosphorylated p38. In this case, pemetrexed reduces p38 activation similarly to at two different concentration (10 µM and 50 µM). The example liposome reduces phosphorylated p38 more substantially demonstrating that the pemetrexed inside the example liposome is active (Figure 19).

Another series of experiments was conducted to show that the example liposome inhibits cellular proliferation in similar degree to free pemetrexed at matched concentrations as pemetrexed is an effective chemotherapy in stopping cancer cells from dividing. By inhibiting the formation of precursor purine and pyrimidine nucleotides, pemetrexed prevents the formation of DNA and RNA, which are required for the growth and survival of both normal cells and cancer cells. This was done in two ways:
First, we tested whether the pemetrexed contained within the example liposome was effective in inhibiting cells from dividing, also referred to as cell proliferation. Several cancer cell lines were treated with either 10 mM pemetrexed or the example liposome with an estimated matched concentration of pemetrexed for four days. The cells were then assessed for numbers of cells that divided. The results demonstrated that there is a correlation between cell growth inhibition and folate receptor alpha expression on the cancer cell surface (Figure 20). The results further showed that not only was there a susceptibility of folate receptor alpha expressing cancer cells to the example liposome but also that the example liposome and pemetrexed have similar effects on each of the cell lines (Figure 21).

To further assess the effect of the example liposome on cell cycle, a second approach was used to measure the effects of pemetrexed on the ability of cells to divide. The rationale was that cells treated with pemetrexed cannot produce new DNA and become trapped in the S phase of the cell cycle (Figures 22a and 22b). Cell lines derived from patient with lung or breast cancer were treated with titrated concentrations of pemetrexed or the example liposome. Cells were incubated for 90 hours at 37 degrees Celsius and cellular viability and number were assessed by MTS. Cells were lysed and the DNA labeled with propidium iodine to quantify cell cycle (Figure 23a). The data showed that pemetrexed treatment induces cells to accumulate in S phase (Figure 23b). Furthermore, SC0003 cells (lung adenocarcinoma) were seeded in 96 well plates and treated the next day with titrated concentrations of example liposome or pemetrexed. On day 5, the cells were fixed and lysed and the DNA was labeled with Propidium Iodide to evaluate cell cycle. The results demonstrated that the example liposome induces the same effect on cell cycle as pemetrexed on each of the cell lines as measured by accumulation of cells in S phase (Figure 24).

Another experiment was conducted to assess the impact of the example liposome on bone marrow cells. The rationale is that one of the side effects from an antifolate treatment, such as pemetrexed containing treatment, is the reduction of neutrophils in the bloodstream, leading to severe and sometime life threatening infections. This is due to CD34+ stem cells not differentiating, or developing, into mature neutrophils in the bone marrow. We measured the effect of the example liposome on neutrophil differentiation compared to the same dose of pemetrexed (10 mM) Stem cells from four human donors were purchased and treated with a panel of growth factors to induce neutrophil differentiation. More specifically, CD34+ stem cells were induced to differentiate into neutrophils with IL-3, stem cell factor, and G-CSF. Cells were treated with 10 mM pemetrexed for two days or with a calculated estimation of the example liposome at 10 mM pemetrexed for two days. Numbers of differentiated neutrophils were assessed by flow cytometry.

The results showed that in absence of pemetrexed, there is a dramatic increase in mature neutrophils by day 2, as depicted in the oval area of Figure 25 and Figure 26A. In the presence of pemetrexed (2-50 mM), however, neutrophil differentiation is inhibited (Figure 26B; n=4 donors) demonstrating that CD34+ stem cells treated with pemetrexed failed to develop into mature neutrophils. In contrast to free premetrexed, the example liposome was able to reduce this toxicity by allowing more CD34+ stem cells to develop and mature into differentiated neutrophils when compared to pemetrexed at similar pemetrexed concentration (Figure 27).

Taken together, these results from the experiments conducted provide evidence that the technology used in the design construct of the example liposome is such that the example liposome as a delivery system of a bioactive agent/payload, armed with a folate receptor targeting moiety other than folic acid or its analogues, enters tumor cells expressing folate receptor alpha the cells regardless of its liposome bioactive agent payload, preserves efficacy of the bioactive agent in folate receptor alpha expressing cancer cells and minimizes exposure of normal cells to the toxic effects of an antifolate agent payload such as a pemetrexed payload, thereby offering the opportunity to reintroduce in the clinical setting otherwise very efficacious but toxic agents, such as antifolates as a class, without typically associated severe and sometime life-threatening toxicities.

## Claims

1. A liposomal antifolate composition comprising:
a liposome including an interior space;
a bioactive antifolate agent disposed within said interior space;
a PEG attached to an exterior of the liposome;
a protein with specific affinity for at least one folate receptor, said protein attached to at least one of the PEG and the exterior of the liposome; and
wherein the liposome has a zeta potential that is less than or equal to zero and the liposome is anionic or neutral.

2. The liposomal antifolate composition of claim 1, wherein said PEG has a number average molecular weight (Mn) of 200 to 5000 daltons.

3. The liposomal antifolate composition of claim 1, further comprising at least one of an immunostimulatory agent and a maleimide disposed on at least one of the PEG and an exterior of the liposome wherein the at least one immunostimulatory agent is:
covalently bonded to at least one of the PEG and the exterior of the liposome;
is selected from a hapten and an adjuvant;
fluorescein isothiocyanate (FITC); or
at least one selected from the group consisting of: fluorescein; DNP; beta glucan; beta-1,3-glucan; and beta-1,6-glucan.

4. The liposomal antifolate composition of claim 1, wherein the liposome has a diameter in the range of 30-150 nm, preferably 40-70 nm.

5. The liposomal antifolate composition of claim 1, wherein the zeta potential of the liposome is in a range of 0 to -150 mV or wherein the zeta potential of the liposome is in a range of -30 to -50 mV.

6. The liposomal antifolate composition of claim 1 wherein the liposome is formed from liposomal components comprising:
at least one of an anionic lipid and a neutral lipid;
at least one selected from the group consisting of: DSPE; DSPE-PEG-maleimide; HSPC; HSPC-PEG; cholesterol; cholesterol-PEG; and cholesterol-maleimide; or
at least one selected from the group consisting of: DSPE; DSPE-PEG-FITC; DSPE-PEG-maleimide; cholesterol; and HSPC.

7. The liposomal antifolate composition of claim 1 wherein the liposome encloses an aqueous solution.

8. The liposomal antifolate composition of claim 1 wherein the liposome encloses a bioactive antifolate agent and an aqueous pharmaceutically acceptable carrier;
optionally wherein the pharmaceutically acceptable carrier comprises trehalose;
optionally wherein the pharmaceutically acceptable carrier comprises 5% to 20% weight percent of trehalose;
optionally wherein the pharmaceutically acceptable carrier comprises citrate buffer at a concentration of between 5 to 200 mM and a pH of between 2.8 to 6; or optionally wherein the pharmaceutically acceptable carrier comprises a total concentration of sodium acetate and calcium acetate of between 50 mM to 500 mM.

9. The liposomal antifolate composition of claim 1 wherein the bioactive antifolate agent is water soluble.

10. The liposomal antifolate composition of claim 1 wherein each liposome comprises less than 200,000 molecules of the bioactive antifolate agent; or between 10,000 to 100,000 molecules of the bioactive antifolate agent.

11. The liposomal antifolate composition of claim 1 wherein the bioactive antifolate agent is:
pemetrexed:
lometrexol;
at least one selected from the group consisting of methotrexate; ralitrexed;
aminopterin; pralatrexate; lometrexol; thiophene analog of lometrexol; furan
analog of lometrexol; trimetrexed; LY309887; and GW 1843U89; or
at least one selected from the group consisting of proguanil; pyrimethamine;
trimethoprim; and 6-Substituted Pyrrolo and a member of the Thieon[2,3-d]pyrrolopyrimidine class of GARFT inhibitors.

12. The liposomal antifolate composition of claim 1 wherein the bioactive antifolate agent is at a pH of 5-8; or at a pH of 2-6.

13. The liposomal antifolate composition of claim 1 wherein the protein is covalently bound via a maleimide functional group to a PEG molecule.

14. The liposomal antifolate composition of claim 1 wherein the protein has specific affinity for at least one, at least two, or all three selected from the group consisting of: folate receptor alpha; folate receptor beta; and folate receptor delta.

15. The liposomal antifolate composition of claim 1 wherein the protein has specific affinity for an epitope on a tumor cell surface antigen that is present on a tumor cell but absent or inaccessible on a non-tumor cell;
optionally wherein said tumor cell is a malignant cell; or
optionally wherein the tumor cell surface antigen is at least one selected from the group consisting of: folate receptor alpha; folate receptor beta; and folate receptor delta.

16. The liposomal antifolate composition of claim 1 wherein the protein comprises an antigen binding sequence of an antibody;
optionally wherein the antigen binding sequence of an antibody comprises one or more complementarity determining regions of antibody origin; or
optionally wherein the protein is at least one selected from the group consisting of an antibody; a humanized antibody; an antigen binding fragment of an antibody; a single chain antibody; a single-domain antibody; a bi-specific antibody; a synthetic antibody; a pegylated antibody; and a multimeric antibody.

17. The liposomal antifolate composition of claim 1 wherein each liposome comprises up to 200 of the proteins; optionally from 30 to 200 of the proteins.

18. The liposomal antifolate composition of any of claims 1-17 for use in the treatment of cancer.

19. A method of preparing a composition of claim 6 comprising:
forming a mixture comprising:
liposomal components comprising at least one of: DSPE; DSPE-PEG-maleimide; HSPC; HSPC-PEG; cholesterol; cholesterol-PEG; and cholesterol-maleimide; or at least one of: DSPE; DSPE-PEG-FITC; DSPE-PEG-maleimide; cholesterol; and HSPC;
the bioactive antifolate agent in aqueous solution;
the protein;
homogenizing the mixture to form liposomes in said aqueous solution;
extruding the mixture through a membrane to form liposomes enclosing the bioactive antifolate agent in an aqueous solution;
optionally removing excess bioactive antifolate agent in aqueous solution outside of the liposomes after said extruding step, further optionally lyophilizing said composition after said removing step to form a lyophilized composition, further optionally reconstituting said lyophilizing composition by dissolving said lyophilizing composition in a solvent after said lyophilizing step, and further optionally wherein said solvent is an aqueous solvent; and
optionally wherein the mixture comprises at least one selected from the group consisting of mannitol; trehalose; sorbitol; and sucrose.

20. The method of claim 19 wherein the liposome further comprises a steric stabilizer; wherein the steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[*N*-(2-hydroxypropyl) methacrylamide]; amphiphilic poly-*N*-vinylpyrrolidones; L-amino-acid-based polymer; and polyvinyl alcohol; and optionally wherein the at least one stabilizer is PEG, and the PEG has a number average molecular weight (Mn) of 200 to 5000 daltons.

21. A liposomal antifolate composition comprising:
a medium comprising a liposome including an interior space;
an aqueous bioactive antifolate agent disposed within said interior space;
a protein with specific affinity for at least one folate receptor, said protein disposed at an the exterior of the liposome;
wherein the liposome has a zeta potential that is less than or equal to zero and the liposome is anionic or neutral; optionally wherein the medium is an aqueous solution; optionally wherein the aqueous solution comprises at least one cryoprotectant selected from the group consisting of mannitol; trehalose; sorbitol; and sucrose;
optionally wherein the liposomal antifolate composition further comprises a steric stabilizer attached to the exterior of the liposome, wherein the protein is attached to at least one of the steric stabilizer and the exterior of the liposome,-and optionally wherein the steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[*N*-(2-hydroxypropyl) methacrylamide]; amphiphilic poly-*N-*vinylpyrrolidones; L-amino-acid-based polymer; and polyvinyl alcohol; and further optionally wherein the steric stabilizer is PEG and the PEG has a number average molecular weight (Mn) of 200 to 5000 daltons.

22. The liposomal antifolate composition of claim 21 further comprising at least one of an immunostimulatory agent and a maleimide disposed on at least one of the steric stabilizer and an exterior of the liposome; wherein the at least one immunostimulatory agent is covalently bonded to at least one of the steric stabilizer and the exterior of the liposome; and the at least one immunostimulatory agent is selected from a hapten and an adjuvant; fluorescein isothiocyanate (FITC); or at least one selected from the group consisting of: fluorescein; DNP; beta glucan; beta-1,3-glucan; and beta-1,6-glucan.

23. The liposomal antifolate composition of claim 21 wherein the liposome has a diameter in the range of 30-150 nm; preferably in the range of 40-70 nm.

24. The liposomal antifolate composition of claim 21 wherein the zeta potential of the liposome is in a range of 0 to -150 mV or wherein the zeta potential of the liposome is in the range of -30 to -50 mV.

25. The liposomal antifolate composition of claim 21 wherein the liposome is formed from liposomal components comprising:
at least one of an anionic lipid and a neutral lipid;
at least one selected from the group consisting of: DSPE; DSPE-PEG; DSPE-maleimide; HSPC; HSPC-PEG; HSPC-maleimide; cholesterol; cholesterol-PEG; and cholesterol-maleimide; or
at least one selected from the group consisting of: DSPE; DSPE-FITC; DSPE-maleimide; cholesterol; and HSPC.

26. The liposomal antifolate composition of claim 21 wherein the liposome encloses an aqueous solution;
optionally wherein the liposome encloses a bioactive antifolate agent and an aqueous pharmaceutically acceptable carrier, optionally wherein the pharmaceutically acceptable carrier comprises trehalose, further optionally wherein the pharmaceutically acceptable carrier comprises 5% to 20% weight percent of trehalose;
optionally wherein the pharmaceutically acceptable carrier comprises citrate buffer at a concentration of between 5 to 200 mM and a pH of between 2.8 to 6; and optionally wherein the pharmaceutically acceptable carrier comprises a total concentration of sodium acetate and calcium acetate of between 50 mM to 500 mM.

27. The liposomal antifolate composition of claim 21 wherein the bioactive antifolate agent is water soluble.

28. The liposomal antifolate composition of claim 21 wherein each liposome comprises less than 200,000 molecules of the bioactive antifolate agent; or between 10,000 to 100,000 of the bioactive antifolate agent.

29. The liposomal antifolate composition of claim 21 wherein the bioactive antifolate agent is
pemetrexed;
lometrexol;
at least one selected from the group consisting of methotrexate; ralitrexed;
aminopterin; pralatrexate; lometrexol; thiophene analog of lometrexol; furan
analog of lometrexol; trimetrexed; LY309887; and GW 1843U89; or
at least one selected from the group consisting of proguanil; pyrimethamine; trimethoprim; and 6-Substituted Pyrrolo and a member of the Thieon[2,3-d]pyrrolopyrimidine class of GARFT inhibitors.

30. The liposomal antifolate composition of claim 21 wherein the bioactive antifolate agent is at a pH of 5-8 or at a pH of 2-6.

31. The liposomal antifolate composition of claim 21 wherein the protein is covalently bound via a maleimide functional group to a steric stabilizer molecule.

32. The liposomal antifolate composition of claim 21 wherein the protein has specific affinity for at least one, at least two, or all three selected from the group consisting of: folate receptor alpha; folate receptor beta; and folate receptor delta.

33. The liposomal antifolate composition of claim 21 wherein the protein has specific affinity for an epitope on a tumor cell surface antigen that is present on a tumor cell but absent or inaccessible on a non-tumor cell;
optionally wherein said tumor cell is a malignant cell; or
optionally wherein the tumor cell surface antigen is at least one selected from the group consisting of: folate receptor alpha; folate receptor beta; and folate receptor delta.

34. The liposomal antifolate composition of claim 21 wherein the protein comprises an antigen binding sequence of an antibody;
optionally wherein the antigen binding sequence of an antibody comprises one or more complementarity determining regions of antibody origin; or
optionally wherein the protein is at least one selected from the group consisting of an antibody; a humanized antibody; an antigen binding fragment of an antibody; a single chain antibody; a single-domain antibody; a bi-specific antibody; a synthetic antibody; a pegylated antibody; and a multimeric antibody.

35. The liposomal antifolate composition of claim 21 wherein each liposome comprises up to 200 targeting moieties; optionally each liposome comprises from 30 to 200 targeting moieties.

36. The liposomal antifolate composition of claim 21 for use in the treatment of cancer.

37. A method of preparing a composition of claim 25 comprising:
forming a mixture comprising:
liposomal components comprising at least one selected from the group consisting of:
DSPE; DSPE-PEG; DSPE-maleimide; HSPC; HSPC-PEG; HSPC-maleimide;
cholesterol; cholesterol-PEG; and cholesterol-maleimide; and
at least one selected from the group consisting of: DSPE; DSPE-FITC; DSPE-maleimide; cholesterol; and HSPC;
the bioactive antifolate agent in aqueous solution;
the protein;
homogenizing the mixture to form liposomes in said aqueous solution; and
extruding the mixture through a membrane to form liposomes enclosing the bioactive antifolate agent in an aqueous solution;
optionally removing excess bioactive antifolate agent in aqueous solution outside of the liposomes after said extruding step, further optionally lyophilizing said composition after said removing step to form a lyophilized composition, and further optionally reconstituting said lyophilizing composition by dissolving said lyophilizing composition in a solvent after said lyophilizing step, and further optionally wherein said solvent is an aqueous solvent; and
optionally wherein the mixture comprises at least one selected from the group consisting of mannitol; trehalose; sorbitol; and sucrose.

38. The method of claim 37 wherein the formed liposomes further comprise a steric stabilizer wherein the steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[*N*-(2-hydroxypropyl) methacrylamide]; amphiphilic poly-*N*-vinylpyrrolidones; L-amino-acid-based polymer; and polyvinyl alcohol; and optionally wherein the at least one stabilizer is PEG and said PEG has a number average molecular weight (Mn) of 200 to 5000 daltons.

39. A targeted liposomal composition that selectively targets folate receptors comprising:
a liposome including an interior space;
a bioactive agent disposed within said interior space;
a steric stabilizer molecule attached to an exterior of the liposome; and
a protein with specific affinity for at least one folate receptor, said protein attached to at least one of the steric stabilizer and the exterior of the liposome;
wherein the liposome has a zeta potential that is less than or equal to zero and the liposome is anionic or neutral;
optionally wherein the steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[*N*-(2-hydroxypropyl) methacrylamide]; amphiphilic poly-*N-*vinylpyrrolidones; L-amino-acid-based polymer; and polyvinyl alcohol, optionally wherein when the steric stabilizer is PEG and the PEG has a number average molecular weight (Mn) of 200 to 5000 daltons; and
optionally wherein the bioactive agent comprises at least one of the group consisting of ellipticine; paclitaxel; pemetrexed; methotrexate; ralitrexed; aminopterin; pralatrexate; lometrexol; trimetrexed; LY309887; GW 1843U89; proguanil; pyrimethamine; trimethoprim and 6-Substituted Pyrrolo and Thieon[2,3-d]pyrrolopyrimidine class of GARFT inhibitors.

40. The liposomal antifolate composition of claim 39 for use in the treatment of cancer.

41. A method of preparing a composition of claim 39 wherein said liposome is formed comprising the steps of:
forming a mixture comprising:
liposomal components comprising at least one selected from the group consisting of: DSPE; DSPE-PEG; DSPE-maleimide; HSPC; HSPC-PEG; HSPC-maleimide; cholesterol; cholesterol-PEG; and cholesterol-maleimide; and at least one selected from the group consisting of: DSPE; DSPE-FITC; DSPE-maleimide; cholesterol; and HSPC;
the bioactive agent in aqueous solution;
the protein;
homogenizing the mixture to form liposomes in said aqueous solution; and
extruding the mixture through a membrane to form liposomes enclosing the bioactive antifolate agent in an aqueous solution;
optionally removing excess bioactive antifolate agent in aqueous solution outside of the liposomes after said extruding step, further optionally lyophilizing said composition after said removing step to form a lyophilized composition, and further optionally reconstituting said lyophilizing composition by dissolving said lyophilizing composition in a solvent after said lyophilizing step, and further optionally wherein said solvent is an aqueous solvent; and
optionally wherein the mixture comprises at least one selected from the group consisting of mannitol; trehalose; sorbitol; and sucrose.

42. The method of claim 41 wherein the formed liposomes further comprises a steric stabilizer; wherein the steric stabilizer is at least one selected from the group consisting of polyethylene glycol (PEG); poly-L-lysine (PLL); monosialoganglioside (GM1); poly(vinyl pyrrolidone) (PVP); poly(acrylamide) (PAA); poly(2-methyl-2-oxazoline); poly(2-ethyl-2-oxazoline); phosphatidyl polyglycerol; poly[*N*-(2-hydroxypropyl) methacrylamide]; amphiphilic poly-*N*-vinylpyrrolidones; L-amino-acid-based polymer; and polyvinyl alcohol; and optionally wherein the at least one stabilizer is PEG and the PEG has a number average molecular weight (Mn) of 200 to 5000 daltons.

43. A kit for providing a composition of claim 25 comprising:
liposomal components comprising at least one selected from the group consisting of:
DSPE; DSPE-PEG; DSPE-maleimide; HSPC; HSPC-PEG; HSPC-maleimide;
cholesterol; cholesterol-PEG; and cholesterol-maleimide; and at least one selected from the group consisting of: DSPE; DSPE-FITC; DSPE-maleimide; cholesterol;
and HSPC,
an instruction for using the composition to encapsulate a bioactive agent, and
optionally, in a separate container, the bioactive agent.

## Patentansprüche

1. Liposomale Antifolat-Zusammensetzung, umfassend:
ein Liposom, welches einen Innenraum umfasst;
ein bioaktives Antifolat-Agens, welches in dem Innenraum angeordnet ist;
ein PEG, welches an einer Außenseite des Liposoms befestigt ist;
ein Protein mit spezifischer Affinität für mindestens einen Folatrezeptor, wobei das Protein an mindestens einem aus dem PEG und der Außenseite des Liposoms befestigt ist; und
wobei das Liposom ein Zeta-Potential aufweist, das kleiner oder gleich Null ist, und das Liposom anionisch oder neutral ist.

2. Liposomale Antifolat-Zusammensetzung nach Anspruch 1, wobei das PEG ein Zahlenmittel-Molekulargewicht (Mn) von 200 bis 5000 Dalton aufweist.

3. Liposomale Antifolat-Zusammensetzung nach Anspruch 1, ferner umfassend mindestens eines aus einem immunstimulierenden Agens und einem Maleimid, angeordnet auf mindestens einem aus dem PEG und einer Außenseite des Liposoms, wobei das mindestens eine immunstimulierende Agens:
kovalent an mindestens eines aus dem PEG und der Außenseite des Liposoms gebunden ist;
aus einem Hapten und einem Adjuvans ausgewählt ist;
Fluoresceinisothiocyanat (FITC) ist; oder
mindestens eines ist, ausgewählt aus der Gruppe, bestehend aus: Fluorescein; DNP; Betaglucan, Beta-1,3-glucan und Beta-1,6-glucan.

4. Liposomale Antifolat-Zusammensetzung nach Anspruch 1, wobei das Liposom einen Durchmesser im Bereich von 30 nm bis 150 nm, vorzugsweise von 40 nm bis 70 nm aufweist.

5. Liposomale Antifolat-Zusammensetzung nach Anspruch 1, wobei das Zeta-Potential des Liposoms in einem Bereich von 0 mV bis -150 mV liegt oder wobei das Zeta-Potential des Liposoms in einem Bereich von -30 mV bis -50 mV liegt.

6. Liposomale Antifolat-Zusammensetzung nach Anspruch 1, wobei das Liposom aus liposomalen Komponenten ausgebildet ist, umfassend:
mindestens ein anionisches Lipid und ein neutrales Lipid;
mindestens eines, ausgewählt aus der Gruppe, bestehend aus: DSPE; DSPE-PEG-Maleimid; HSPC; HSPC-PEG; Cholesterol; Cholesterol-PEG und Cholesterol-Maleimid; oder
mindestens eines, ausgewählt aus der Gruppe, bestehend aus: DSPE; DSPE-PEG-FITC; DSPE-PEG-Maleimid; Cholesterol und HSPC.

7. Liposomale Antifolat-Zusammensetzung nach Anspruch 1, wobei das Liposom eine wässrige Lösung umschließt.

8. Liposomale Antifolat-Zusammensetzung nach Anspruch 1, wobei das Liposom ein bioaktives Antifolat-Agens und einen wässrigen pharmazeutisch akzeptablen Träger umschließt;
wobei der pharmazeutisch akzeptable Träger gegebenenfalls Trehalose umfasst;
wobei der pharmazeutisch akzeptable Träger gegebenenfalls 5 bis 20 Gewichts-% Trehalose umfasst;
wobei der pharmazeutisch akzeptable Träger gegebenenfalls Citratpuffer in einer Konzentration von 5 mM bis 200 mM und einen pH-Wert von 2,8 bis 6 umfasst; oder
wobei der pharmazeutisch akzeptable Träger gegebenenfalls eine Gesamtkonzentration von Natriumacetat und Calciumacetat von 50 mM bis 500 mM umfasst.

9. Liposomale Antifolat-Zusammensetzung nach Anspruch 1, wobei das bioaktive Antifolat-Agens wasserlöslich ist.

10. Liposomale Antifolat-Zusammensetzung nach Anspruch 1, wobei jedes Liposom weniger als 200.000 Moleküle des bioaktiven Antifolat-Agens oder 10.000 bis 100.000 Moleküle des bioaktiven Antifolat-Agens umfasst.

11. Liposomale Antifolat-Zusammensetzung nach Anspruch 1, wobei das bioaktive Antifolat-Agens Folgendes ist:
Pemetrexed;
Lometrexol;
mindestens eines, ausgewählt aus der Gruppe, bestehend aus Methotrexat; Ralitrexed; Aminopterin; Pralatrexat; Lometrexol; Thiophen-Analog von Lometrexol; Trimetrexed; LY309887 und GW 1843U89; oder
mindestens eines, ausgewählt aus der Gruppe, bestehend aus Proguanil; Pyrimethamin; Trimethoprim und 6-substituiertem Pyrrolo und einem Element der Thienon[2,3-d]pyrrolopyrimidin-Klasse der GARFT-Inhibitoren.

12. Liposomale Antifolat-Zusammensetzung nach Anspruch 1, wobei das bioaktive Antifolat-Agens einen pH-Wert von 5 bis 8 aufweist oder einen pH-Wert von 2 bis 6 aufweist.

13. Liposomale Antifolat-Zusammensetzung nach Anspruch 1, wobei das Protein über eine funktionelle Maleimid-Gruppe kovalent an ein PEG-Molekül gebunden ist.

14. Liposomale Antifolat-Zusammensetzung nach Anspruch 1, wobei das Protein eine spezifische Affinität für mindestes eines, mindestens zwei oder alles drei aufweist, ausgewählt aus der Gruppe, bestehend aus: Folatrezeptor Alpha; Folatrezeptor Beta und Folatrezeptor Delta.

15. Liposomale Antifolat-Zusammensetzung nach Anspruch 1, wobei das Protein eine spezifische Affinität für ein Epitop auf einem Tumorzellen-Oberflächenantigen aufweist, welches auf einer Tumorzelle vorliegt, aber auf einer Nicht-Tumorzelle nicht vorliegt oder nicht zugänglich ist;
wobei die Tumorzelle gegebenenfalls eine bösartige Zelle ist; oder
wobei das Tumorzellen-Oberflächenantigen gegebenenfalls mindestens eines ist, ausgewählt aus der Gruppe, bestehend aus: Folatrezeptor Alpha, Folatrezeptor Beta und Folatrezeptor Delta.

16. Liposomale Antifolat-Zusammensetzung nach Anspruch 1, wobei das Protein eine Antigen-Bindesequenz eines Antikörpers umfasst;
wobei die Antigen-Bindesequenz eines Antikörpers gegebenenfalls eine oder mehrere Komplementaritäts-Bestimmungszonen mit Antikörper-Ursprung umfasst; oder
wobei das Protein gegebenenfalls mindestens eines ist, ausgewählt aus der Gruppe, bestehend aus einem Antikörper; einem humanisierten Antikörper; einem Antigen-Bindefragment eines Antikörpers; einem einkettigen Antikörper; einem Einzeldomänen-Antikörper; einem bispezifischen Antikörper; einem synthetischen Antikörper; einem pegylierten Antikörper und einem multimeren Antikörper.

17. Liposomale Antifolat-Zusammensetzung nach Anspruch 1, wobei jedes Liposom bis zu 200 der Proteine umfasst; gegebenenfalls 30 bis 200 der Proteine umfasst.

18. Liposomale Antifolat-Zusammensetzung nach einem der Ansprüche 1 bis 17 zur Verwendung bei der Behandlung einer Krebserkrankung.

19. Verfahren zur Herstellung einer Zusammensetzung nach Anspruch 6, umfassend:
Bilden eines Gemisches, umfassend:
liposomale Komponenten, umfassend mindestens eines aus: DSPE; DSPE-PEG-Maleimid; HSPC; HSPC-PEG; Cholesterol; Cholesterol-PEG und Cholesterol-Maleimid; oder mindestens eines, ausgewählt aus der Gruppe, bestehend aus: DSPE; DSPE-PEG-FITC; DSPE-PEG-Maleimid; Cholesterol und HSPC;
das bioaktive Antifolat-Agens in wässriger Lösung;
das Protein;
Homogenisieren des Gemisches, um Liposome in der wässrigen Lösung zu bilden;
Extrudieren des Gemisches durch eine Membran, um Liposome zu bilden, welche das bioaktive Antifolat-Agens in einer wässrigen Lösung umschließen;
gegebenenfalls Entfernen von überschüssigem bioaktivem Antifolat-Agens in wässriger Lösung außerhalb der Liposome nach dem Schritt des Extrudierens, ferner gegebenenfalls Gefriertrocknen der Zusammensetzung nach dem Schritt des Entfernens, um eine gefriergetrocknete Zusammensetzung zu bilden, ferner gegebenenfalls Rekonstituieren der Gefriertrocknungszusammensetzung durch Lösen der Gefriertrocknungszusammensetzung in einem Lösungsmittel nach dem Schritt des Gefriertrocknens und wobei ferner das Lösungsmittel gegebenenfalls ein wässriges Lösungsmittel ist; und
wobei das Gemisch gegebenenfalls mindestens eines umfasst, ausgewählt aus der Gruppe, bestehend aus Mannitol; Trehalose; Sorbitol und Saccharose.

20. Verfahren nach Anspruch 19, wobei das Liposom ferner einen sterischen Stabilisator umfasst, wobei der sterische Stabilisator mindestens eines ist, ausgewählt aus der Gruppe, bestehend aus Polyethylenglykol (PEG); Poly-L-Lysin (PLL); Monosialogangliosid (GM1); Polyvinylpyrrolidon (PVP); Polyacrylamid (PAA); Poly(2-methyl-2-oxazolin); Poly(2-ethyl-2-oxazolin); Phosphatidylpolyglycerol; Poly[N-(2-hydroxypropyl)methacrylamid]; amphiphilen Poly-N-vinylpyrrolidonen; Polymer auf L-AminosäureBasis und Polyvinylalkohol; und wobei gegebenenfalls der mindestens eine Stabilisator PEG ist und das PEG ein Zahlenmittel-Molekulargewicht (Mn) von 200 bis 5.000 Dalton aufweist.

21. Liposomale Antifolat-Zusammensetzung, umfassend:
ein Medium, welches ein Liposom umfasst, das einen Innenraum umfasst;
ein wässriges bioaktives Antifolat-Agens, welches in dem Innenraum angeordnet ist;
ein Protein mit spezifischer Affinität für mindestens einen Folatrezeptor, wobei das Protein an der Außenseite des Liposoms angeordnet ist;
wobei das Liposom ein Zeta-Potential aufweist, das kleiner oder gleich Null ist, und das Liposom anionisch oder neutral ist; wobei das Medium gegebenenfalls eine wässrige Lösung ist; wobei die wässrige Lösung gegebenenfalls mindestens ein Gefrierschutzmittel umfasst, ausgewählt aus der Gruppe, bestehend aus Mannitol; Trehalose; Sorbitol und Saccharose;
wobei die liposomale Antifolat-Zusammensetzung ferner gegebenenfalls einen sterischen Stabilisator umfasst, der an der Außenseite des Liposoms befestigt ist, wobei das Protein an mindestens einem aus dem sterischen Stabilisator und der Außenseite des Liposoms befestigt ist, und wobei der sterische Stabilisator gegebenenfalls mindestens eines ist, ausgewählt aus der Gruppe, bestehend aus Polyethylenglykol (PEG); Poly-L-Lysin (PLL); Monosialogangliosid (GM1); Polyvinylpyrrolidon (PVP); Polyacrylamid (PAA); Poly(2-methyl-2-oxazolin); Poly(2-ethyl-2-oxazolin); Phosphatidylpolyglycerol; Poly[N-(2-hydroxypropyl)methacrylamid]; amphiphilen Poly-N-vinylpyrrolidonen; Polymer auf L-AminosäureBasis und Polyvinylalkohol; und wobei gegebenenfalls der mindestens eine Stabilisator PEG ist und das PEG ein Zahlenmittel-Molekulargewicht (Mn) von 200 bis 5.000 Dalton aufweist.

22. Liposomale Antifolat-Zusammensetzung nach Anspruch 21, ferner umfassend mindestens eines aus einem immunstimulierenden Agens und einem Maleimid, das auf mindestens einem aus dem sterischen Stabilisator und der Außenseite des Liposoms angeordnet ist; wobei das mindestens eine immunstimulierende Agens kovalent an mindestens eines aus dem sterischen Stabilisator und der Außenseite des Liposoms gebunden ist und das mindestens eine immunstimulierende Agens aus einem Hapten und einem Adjuvans ausgewählt ist; Fluoresceinisothiocyanat (FITC) ist; oder mindestens eines ist, ausgewählt aus der Gruppe, bestehend aus: Fluorescein; DNP; Betaglucan; Beta-1,3-glucan und Beta-1,6-glucan.

23. Liposomale Antifolat-Zusammensetzung nach Anspruch 21, wobei das Liposom einen Durchmesser im Bereich von 30 nm bis 150 nm, vorzugsweise im Bereich von 40 nm bis 70 nm aufweist.

24. Liposomale Antifolat-Zusammensetzung nach Anspruch 21, wobei das Zeta-Potential des Liposoms in einem Bereich von 0 mV bis -150 mV liegt oder wobei das Zeta-Potential des Liposoms im Bereich von -30 mV bis - 50 mV liegt.

25. Liposomale Antifolat-Zusammensetzung nach Anspruch 21, wobei das Liposom aus liposomalen Komponenten gebildet ist, umfassend:
mindestens eines aus einem anionischen Lipid und einem neutralen Lipid;
mindestens eines, ausgewählt aus der Gruppe, bestehend aus: DSPE; DSPE-PEG; DSPE-Maleimid; HSPC; HSPC-PEG; HSPC-Maleimid; Cholesterol; Cholesterol-PEG und Cholesterol-Maleimid; oder
mindestens eines, ausgewählt aus der Gruppe, bestehend aus: DSPE; DSPE-PEG-FITC; DSPE-PEG-Maleimid; Cholesterol und HSPC.

26. Liposomale Antifolat-Zusammensetzung nach Anspruch 21, wobei das Liposom eine wässrige Lösung umschließt;
wobei das Liposom gegebenenfalls ein bioaktives Antifolat-Agens und einen wässrigen pharmazeutisch akzeptablen Träger umschließt;
wobei der pharmazeutisch akzeptable Träger gegebenenfalls Trehalose umfasst;
wobei der pharmazeutisch akzeptable Träger ferner gegebenenfalls 5 bis 20 Gewichts-% Trehalose umfasst;
wobei der pharmazeutisch akzeptable Träger gegebenenfalls Citratpuffer in einer Konzentration von 5 mM bis 200 mM und einen pH-Wert von 2,8 bis 6 umfasst; oder
wobei der pharmazeutisch akzeptable Träger gegebenenfalls eine Gesamtkonzentration von Natriumacetat und Calciumacetat von 50 mM bis 500 mM umfasst.

27. Liposomale Antifolat-Zusammensetzung nach Anspruch 21, wobei das bioaktive Antifolat-Agens wasserlöslich ist.

28. Liposomale Antifolat-Zusammensetzung nach Anspruch 21, wobei jedes Liposom weniger als 200.000 Moleküle des bioaktiven Antifolat-Agens oder 10.000 bis 100.000 des bioaktiven Antifolat-Agens umfasst.

29. Liposomale Antifolat-Zusammensetzung nach Anspruch 21, wobei das bioaktive Antifolat-Agens Folgendes ist:
Pemetrexed;
Lometrexol;
mindestens eines, ausgewählt aus der Gruppe, bestehend aus Methotrexat; Ralitrexed; Aminopterin; Pralatrexat; Lometrexol; Thiophen-Analog von Lometrexol; Trimetrexed; LY309887 und GW 1843U89; oder
mindestens eines, ausgewählt aus der Gruppe, bestehend aus Proguanil; Pyrimethamin; Trimethoprim und 6-substituiertem Pyrrolo und einem Element der Thienon[2,3-d]pyrrolopyrimidin-Klasse der GARFT-Inhibitoren.

30. Liposomale Antifolat-Zusammensetzung nach Anspruch 21, wobei das bioaktive Antifolat-Agens einen pH-Wert von 5 bis 8 aufweist oder einen pH-Wert von 2 bis 6 aufweist.

31. Liposomale Antifolat-Zusammensetzung nach Anspruch 21, wobei das Protein über eine funktionelle Maleimid-Gruppe kovalent an ein sterisches Stabilisatormolekül gebunden ist.

32. Liposomale Antifolat-Zusammensetzung nach Anspruch 21, wobei das Protein eine spezifische Affinität für mindestes eines, mindestens zwei oder alles drei aufweist, ausgewählt aus der Gruppe, bestehend aus: Folatrezeptor Alpha; Folatrezeptor Beta und Folatrezeptor Delta.

33. Liposomale Antifolat-Zusammensetzung nach Anspruch 21, wobei das Protein eine spezifische Affinität für ein Epitop auf einem Tumorzellen-Oberflächenantigen aufweist, welches auf einer Tumorzelle vorliegt, aber auf einer Nicht-Tumorzelle nicht vorliegt oder nicht zugänglich ist;
wobei die Tumorzelle gegebenenfalls eine bösartige Zelle ist; oder
wobei das Tumorzellen-Oberflächenantigen gegebenenfalls mindestens eines ist, ausgewählt aus der Gruppe, bestehend aus: Folatrezeptor Alpha, Folatrezeptor Beta und Folatrezeptor Delta.

34. Liposomale Antifolat-Zusammensetzung nach Anspruch 21, wobei das Protein eine Antigen-Bindesequenz eines Antikörpers umfasst;
wobei die Antigen-Bindesequenz eines Antikörpers gegebenenfalls eine oder mehrere Komplementaritäts-Bestimmungszonen mit Antikörper-Ursprung umfasst; oder wobei das Protein gegebenenfalls mindestens eines ist, ausgewählt aus der Gruppe, bestehend aus einem Antikörper; einem humanisierten Antikörper; einem Antigen-Bindefragment eines Antikörpers; einem einkettigen Antikörper; einem Einzeldomänen-Antikörper; einem bispezifischen Antikörper; einem synthetischen Antikörper; einem pegylierten Antikörper und einem multimeren Antikörper.

35. Liposomale Antifolat-Zusammensetzung nach Anspruch 21, wobei jedes Liposom bis zu 200 Zieleinheiten umfasst; wobei jedes Liposom gegebenenfalls 30 bis 200 Zieleinheiten umfasst.

36. Liposomale Antifolat-Zusammensetzung nach Anspruch 21 zur Verwendung bei der Behandlung einer Krebserkrankung.

37. Verfahren zur Herstellung einer Zusammensetzung nach Anspruch 25, umfassend:
Bilden eines Gemisches, umfassend:
liposomale Komponenten, umfassend mindestens eines, ausgewählt aus der Gruppe, bestehend aus: DSPE; DSPE-PEG; DSPE-Maleimid; HSPC; HSPC-PEG; HSPC-Maleimid; Cholesterol; Cholesterol-PEG und Cholesterol-Maleimid; und
mindestens eines, ausgewählt aus der Gruppe, bestehend aus: DSPE; DSPE-FITC; DSPE-Maleimid; Cholesterol und HSPC;
das bioaktive Antifolat-Agens in wässriger Lösung;
das Protein;
Homogenisieren des Gemisches, um Liposome in der wässrigen Lösung zu bilden; und
Extrudieren des Gemisches durch eine Membran, um Liposome zu bilden, welche das bioaktive Antifolat-Agens in einer wässrigen Lösung umschließen;
gegebenenfalls Entfernen von überschüssigem bioaktivem Antifolat-Agens in wässriger Lösung außerhalb der Liposome nach dem Schritt des Extrudierens, ferner gegebenenfalls Gefriertrocknen der Zusammensetzung nach dem Schritt des Entfernens, um eine gefriergetrocknete Zusammensetzung zu bilden, ferner gegebenenfalls Rekonstituieren der Gefriertrocknungszusammensetzung durch Lösen der Gefriertrocknungszusammensetzung in einem Lösungsmittel nach dem Schritt des Gefriertrocknens, und wobei ferner das Lösungsmittel gegebenenfalls ein wässriges Lösungsmittel ist; und
wobei das Gemisch gegebenenfalls mindestens eines umfasst, ausgewählt aus der Gruppe, bestehend aus Mannitol; Trehalose; Sorbitol und Saccharose.

38. Verfahren nach Anspruch 37, wobei die gebildeten Liposome ferner einen sterischen Stabilisator umfassen, wobei der sterische Stabilisator mindestens eines ist, ausgewählt aus der Gruppe, bestehend aus Polyethylenglykol (PEG); Poly-L-Lysin (PLL); Monosialogangliosid (GM1); Polyvinylpyrrolidon (PVP); Polyacrylamid (PAA); Poly(2-methyl-2-oxazolin); Poly(2-ethyl-2-oxazolin); Phosphatidylpolyglycerol; Poly[N-(2-hydroxypropyl)methacrylamid]; amphiphilen Poly-N-vinylpyrrolidonen; Polymer auf L-AminosäureBasis und Polyvinylalkohol; und wobei gegebenenfalls der mindestens eine Stabilisator PEG ist und das PEG ein Zahlenmittel-Molekulargewicht (Mn) von 200 bis 5.000 Dalton aufweist.

39. Zielorientierte liposomale Zusammensetzung, welche selektiv auf Folatrezeptoren abzielt, umfassend:
ein Liposom, welches einen Innenraum umfasst;
ein bioaktives Agens, welches in dem Innenraum angeordnet ist;
ein sterisches Stabilisatormolekül, welches an einer Außenseite des Liposoms befestigt ist; und
ein Protein mit spezifischer Affinität für mindestens einen Folatrezeptor, wobei das Protein an mindestens einem aus dem sterischen Stabilisator und der Außenseite des Liposoms angeordnet ist;
wobei das Liposom ein Zeta-Potential aufweist, welches kleiner oder gleich Null ist, und das Liposom anionisch oder neutral ist;
wobei gegebenenfalls der sterische Stabilisator mindestens eines ist, ausgewählt aus der Gruppe, bestehend aus Polyethylenglykol (PEG); Poly-L-Lysin (PLL); Monosialogangliosid (GM1); Polyvinylpyrrolidon (PVP); Polyacrylamid (PAA); Poly(2-methyl-2-oxazolin); Poly(2-ethyl-2-oxazolin); Phosphatidylpolyglycerol; Poly[N-(2-hydroxypropyl)methacrylamid]; amphiphilen Poly-N-vinylpyrrolidonen; Polymer auf L-AminosäureBasis und Polyvinylalkohol; und wobei gegebenenfalls der mindestens eine Stabilisator PEG ist und das PEG ein Zahlenmittel-Molekulargewicht (Mn) von 200 bis 5.000 Dalton aufweist; und wobei das bioaktive Agens gegebenenfalls mindestens aus der Gruppe umfasst, bestehend aus Ellipticin; Paclitaxel; Pemetrexed; Methotrexat; Ralitrexed; Aminopterin; Pralatrexat; Lometrexol; Trimetrexed; LY309887; GW 1843U89; Proguanil; Pyrimethamin; Trimethoprim und 6-substituiertem Pyrrolo und die Thienon[2,3-d]pyrrolopyrimidin-Klasse der GARFT-Inhibitoren.

40. Liposomale Antifolat-Zusammensetzung nach Anspruch 39 zur Verwendung bei der Behandlung einer Krebserkrankung.

41. Verfahren zur Herstellung einer Zusammensetzung nach Anspruch 39, wobei das Bilden des Liposoms die Schritte umfasst:
Bilden eines Gemisches, umfassend:
liposomale Komponenten, umfassend mindestens eines, ausgewählt aus der Gruppe, bestehend aus: DSPE; DSPE-PEG; DSPE-Maleimid; HSPC; HSPC-PEG; HSPC-Maleimid; Cholesterol; Cholesterol-PEG und Cholesterol-Maleimid; und
mindestens eines, ausgewählt aus der Gruppe, bestehend aus: DSPE; DSPE-FITC; DSPE-Maleimid; Cholesterol und HSPC;
das bioaktive Antifolat-Agens in wässriger Lösung;
das Protein;
Homogenisieren des Gemisches, um Liposome in der wässrigen Lösung zu bilden; und
Extrudieren des Gemisches durch eine Membran, um Liposome zu bilden, welche das bioaktive Antifolat-Agens in einer wässrigen Lösung umschließen;
gegebenenfalls Entfernen von überschüssigem bioaktivem Antifolat-Agens in wässriger Lösung außerhalb der Liposome nach dem Schritt des Extrudierens, ferner gegebenenfalls Gefriertrocknen der Zusammensetzung nach dem Schritt des Entfernens, um eine gefriergetrocknete Zusammensetzung zu bilden, ferner gegebenenfalls Rekonstituieren der Gefriertrocknungszusammensetzung durch Lösen der Gefriertrocknungszusammensetzung in einem Lösungsmittel nach dem Schritt des Gefriertrocknens, und wobei ferner das Lösungsmittel gegebenenfalls ein wässriges Lösungsmittel ist; und
wobei das Gemisch gegebenenfalls mindestens eines umfasst, ausgewählt aus der Gruppe, bestehend aus Mannitol; Trehalose; Sorbitol und Saccharose.

42. Verfahren nach Anspruch 41, wobei die gebildeten Liposome ferner einen sterischen Stabilisator umfassen; wobei der sterische Stabilisator mindestens eines ist, ausgewählt aus der Gruppe, bestehend aus Polyethylenglykol (PEG); Poly-L-Lysin (PLL); Monosialogangliosid (GM1); Polyvinylpyrrolidon (PVP); Polyacrylamid (PAA); Poly(2-methyl-2-oxazolin); Poly(2-ethyl-2-oxazolin); Phosphatidylpolyglycerol; Poly[N-(2-hydroxypropyl)methacrylamid]; amphiphilen Poly-N-vinylpyrrolidonen; Polymer auf L-AminosäureBasis und Polyvinylalkohol; und wobei gegebenenfalls der mindestens eine Stabilisator PEG ist und das PEG ein Zahlenmittel-Molekulargewicht (Mn) von 200 bis 5.000 Dalton aufweist.

43. Kit zum Bereitstellen einer Zusammensetzung nach Anspruch 25, umfassend:
liposomale Komponenten, welche mindestens eines umfassen, ausgewählt aus der Gruppe, bestehend aus:
DSPE; DSPE-PEG; DSPE-Maleimid; HSPC; HSPC-PEG; HSPC-Maleimid; Cholesterol; Cholesterol-PEG und Cholesterol-Maleimid; und mindestens eines, ausgewählt aus der Gruppe, bestehend aus: DSPE; DSPE-FITC; DSPE-Maleimid; Cholesterol und HSPC,
eine Anweisung zum Verwenden der Zusammensetzung zum Verkapseln eines bioaktiven Mittels und, gegebenenfalls, in einem separaten Behälter, das bioaktive Agens.

## Revendications

1. Composition antifolate liposomale comprenant:
un liposome incluant un espace intérieur;
un agent antifolate bioactif disposé dans ledit espace intérieur;
un PEG fixé à un extérieur du liposome;
une protéine ayant une affinité spécifique pour au moins un récepteur de folate, ladite protéine fixée à au moins l'un du PEG et de l'extérieur du liposome; et
où le liposome a un potentiel zêta qui est inférieur ou égal à zéro et le liposome est anionique ou neutre.

2. Composition antifolate liposomale selon la revendication 1, où ledit PEG a un poids moléculaire moyen en nombre (Mn) de 200 à 5000 daltons.

3. Composition antifolate liposomale selon la revendication 1, comprenant en outre au moins l'un d'un agent immunostimulant et d'un maléimide disposé sur au moins l'un du PEG et d'un extérieur du liposome, où le au moins un agent immunostimulant est:
lié de manière covalente à au moins l'un du PEG et l'extérieur du liposome;
est choisi parmi un haptène et un adjuvant;
l'isothiocyanate de fluorescéine (FITC); ou
au moins un choisi dans le groupe consistant en: fluorescéine; DNP; bêta-glucane; bêta-1,3-glucane; et bêta-1,6-glucane.

4. Composition antifolate liposomale selon la revendication 1, où le liposome a un diamètre dans la plage de 30 à 150 nm, de préférence de 40 à 70 nm.

5. Composition antifolate liposomale selon la revendication 1, où le potentiel zêta du liposome est dans une plage de 0 à -150 mV ou bien où le potentiel zêta du liposome est dans une plage de -30 à -50 mV.

6. Composition antifolate liposomale selon la revendication 1, où le liposome est formé à partir de composants liposomaux comprenant:
au moins l'un d'un lipide anionique et d'un lipide neutre;
au moins un choisi dans le groupe consistant en: DSPE; DSPE-PEG-maléimide; HSPC; HSPC-PEG; cholestérol; cholestérol-PEG; et cholestérol-maléimide; ou
au moins un choisi dans le groupe consistant en: DSPE; DSPE-PEG-FITC; DSPE-PEG-maléimide; cholestérol; et HSPC.

7. Composition antifolate liposomale selon la revendication 1, où le liposome renferme une solution aqueuse.

8. Composition antifolate liposomale selon la revendication 1, où le liposome renferme un agent antifolate bioactif et un vecteur pharmaceutiquement acceptable aqueux;
facultativement où le vecteur pharmaceutiquement acceptable comprend du tréhalose;
facultativement où le vecteur pharmaceutiquement acceptable comprend 5 % à 20 % en poids de tréhalose;
facultativement où le vecteur pharmaceutiquement acceptable comprend un tampon citrate à une concentration entre 5 et 200 mM et un pH entre 2,8 et 6; ou
facultativement où le vecteur pharmaceutiquement acceptable comprend une concentration totale d'acétate de sodium et d'acétate de calcium entre 50 mM et 500 mM.

9. Composition antifolate liposomale selon la revendication 1, où l'agent antifolate bioactif est soluble dans l'eau.

10. Composition antifolate liposomale selon la revendication 1, où chaque liposome comprend moins de 200 000 molécules de l'agent antifolate bioactif; ou entre 10 000 et 100 000 molécules de l'agent antifolate bioactif.

11. Composition antifolate liposomale selon la revendication 1, où l'agent antifolate bioactif est:
le pémétrexed:
le lométrexol;
au moins un choisi dans le groupe consistant en méthotrexate; ralitrexed; aminoptérine; pralatrexate; lométrexol; analogue thiophène du lométrexol; analogue furane du lométrexol; trimétrexed; LY309887; et GW 1843U89; ou
au moins un choisi dans le groupe consistant en proguanil; pyriméthamine; triméthoprime; et Pyrrolo 6-Substitué et un membre de la classe des Thieon[2,3-d]pyrrolopyrimidines d'inhibiteurs de GARFT.

12. Composition antifolate liposomale selon la revendication 1, où l'agent antifolate bioactif est à un pH de 5 à 8; ou à un pH de 2 à 6.

13. Composition antifolate liposomale selon la revendication 1, où la protéine est liée de manière covalente via un groupe fonctionnel maléimide à une molécule de PEG.

14. Composition antifolate liposomale selon la revendication 1, où la protéine a une affinité spécifique pour au moins un, au moins deux ou tous les trois choisis dans le groupe consistant en: récepteur de folate alpha; récepteur de folate bêta; et récepteur de folate delta.

15. Composition antifolate liposomale selon la revendication 1, où la protéine a une affinité spécifique pour un épitope sur un antigène de surface de cellule tumorale qui est présent sur une cellule tumorale mais absent ou inaccessible sur une cellule non tumorale;
facultativement où ladite cellule tumorale est une cellule maligne; ou
facultativement où l'antigène de surface de cellule tumorale est au moins un choisi dans le groupe consistant en: récepteur de folate alpha; récepteur de folate bêta; et récepteur de folate delta.

16. Composition antifolate liposomale selon la revendication 1, où la protéine comprend une séquence liant un antigène d'un anticorps;
facultativement où la séquence liant un antigène d'un anticorps comprend une ou plusieurs régions déterminant la complémentarité d'origine anticorps; ou
facultativement où la protéine est au moins une choisie dans le groupe consistant en un anticorps; un anticorps humanisé; un fragment liant un antigène d'un anticorps; un anticorps à chaîne unique; un anticorps à domaine unique; un anticorps bispécifique; un anticorps synthétique; un anticorps pégylé; et un anticorps multimère.

17. Composition antifolate liposomale selon la revendication 1, où chaque liposome comprend jusqu'à 200 des protéines; facultativement de 30 à 200 des protéines.

18. Composition antifolate liposomale selon l'une quelconque des revendications 1 à 17 destinée à être utilisée dans le traitement du cancer.

19. Procédé de préparation d'une composition selon la revendication 6 comprenant:
la formation d'un mélange comprenant:
des composants liposomaux comprenant au moins l'un de: DSPE; DSPE-PEG-maléimide; HSPC; HSPC-PEG; cholestérol; cholestérol-PEG; et cholestérol-maléimide; ou au moins l'un de: DSPE; DSPE-PEG-FITC; DSPE-PEG-maléimide; cholestérol; et HSPC;
l'agent antifolate bioactif en solution aqueuse;
la protéine;
l'homogénéisation du mélange pour former des liposomes dans ladite solution aqueuse;
l'extrusion du mélange à travers une membrane pour former des liposomes renfermant l'agent antifolate bioactif dans une solution aqueuse;
facultativement le retrait de l'agent antifolate bioactif en excès en solution aqueuse à l'extérieur des liposomes après ladite étape d'extrusion, en outre facultativement la lyophilisation de ladite composition après ladite étape de retrait pour former une composition lyophilisée, en outre facultativement la reconstitution de ladite composition de lyophilisation par dissolution de ladite composition de lyophilisation dans un solvant après ladite étape de lyophilisation, et en outre facultativement où ledit solvant est un solvant aqueux; et
facultativement où le mélange comprend au moins un choisi dans le groupe consistant en mannitol; tréhalose; sorbitol; et saccharose.

20. Procédé selon la revendication 19, où le liposome comprend en outre un stabilisant stérique; où le stabilisant stérique est au moins un choisi dans le groupe consistant en polyéthylèneglycol (PEG); poly-L-lysine (PLL); monosialoganglioside (GM1); poly(vinylpyrrolidone) (PVP); poly-(acrylamide) (PAA); poly(2-méthyl-2-oxazoline); poly(2-éthyl-2-oxazoline); phosphatidyl polyglycérol; poly[*N*-(2-hydroxypropyl)méthacrylamide]; les poly-*N*-vinylpyrrolidones amphiphiles; un polymère à base de L-amino-acides; et poly(alcool vinylique); et facultativement où le au moins un stabilisant est le PEG, et le PEG a un poids moléculaire moyen en nombre (Mn) de 200 à 5000 daltons.

21. Composition antifolate liposomale comprenant:
un milieu comprenant un liposome incluant un espace intérieur;
un agent antifolate bioactif aqueux disposé dans ledit espace intérieur;
une protéine ayant une affinité spécifique pour au moins un récepteur de folate, ladite protéine disposée à l'extérieur du liposome;
où le liposome a un potentiel zêta qui est inférieur ou égal à zéro et le liposome est anionique ou neutre; facultativement où le milieu est une solution aqueuse; facultativement où la solution aqueuse comprend au moins un cryoprotecteur choisi dans le groupe consistant en mannitol; tréhalose; sorbitol; et saccharose;
facultativement où la composition antifolate liposomale comprend en outre un stabilisant stérique fixé à l'extérieur du liposome, où la protéine est fixée à au moins l'un du stabilisant stérique et de l'extérieur du liposome, et facultativement où le stabilisant stérique est au moins un choisi dans le groupe consistant en polyéthylèneglycol (PEG); poly-L-lysine (PLL); monosialoganglioside (GM1); poly(vinylpyrrolidone) (PVP); poly(acrylamide) (PAA); poly(2-méthyl-2-oxazoline); poly(2-éthyl-2-oxazoline); phosphatidyl polyglycérol; poly[*N-*(2-hydroxypropyl)méthacrylamide]; les poly-*N*-vinylpyrrolidones amphiphiles; un polymère à base de L-aminoacides; et poly(alcool vinylique); et en outre facultativement où le stabilisant stérique est le PEG et le PEG a un poids moléculaire moyen en nombre (Mn) de 200 à 5000 daltons.

22. Composition antifolate liposomale selon la revendication 21, comprenant en outre au moins l'un d'un agent immunostimulant et d'un maléimide disposé sur au moins l'un du stabilisant stérique et d'un extérieur du liposome; où le au moins un agent immunostimulant est lié de manière covalente à au moins l'un du stabilisant stérique et de l'extérieur du liposome; et le au moins un agent immunostimulant est choisi parmi un haptène et un adjuvant; l'isothiocyanate de fluorescéine (FITC); ou au moins un choisi dans le groupe consistant en: fluorescéine; DNP; bêta-glucane; bêta-1,3-glucane; et bêta-1,6-glucane.

23. Composition antifolate liposomale selon la revendication 21, où le liposome a un diamètre dans la plage de 30 à 150 nm; de préférence dans la plage de 40 à 70 nm.

24. Composition antifolate liposomale selon la revendication 21, où le potentiel zêta du liposome est dans une plage de 0 à -150 mV ou bien où le potentiel zêta du liposome est dans la plage de -30 à -50 mV.

25. Composition antifolate liposomale selon la revendication 21, où le liposome est formé à partir de composants liposomaux comprenant:
au moins l'un d'un lipide anionique et d'un lipide neutre;
au moins un choisi dans le groupe consistant en: DSPE; DSPE-PEG; DSPE-maléimide; HSPC; HSPC-PEG; HSPC-maléimide; cholestérol; cholestérol-PEG; et cholestérol-maléimide; ou
au moins un choisi dans le groupe consistant en: DSPE; DSPE-FITC; DSPE-maléimide; cholestérol; et HSPC.

26. Composition antifolate liposomale selon la revendication 21, où le liposome renferme une solution aqueuse;
facultativement où le liposome renferme un agent antifolate bioactif et un vecteur pharmaceutiquement acceptable aqueux, facultativement où le vecteur pharmaceutiquement acceptable comprend du tréhalose, en outre facultativement où le vecteur pharmaceutiquement acceptable comprend 5 % à 20 % en poids de tréhalose;
facultativement où le vecteur pharmaceutiquement acceptable comprend un tampon citrate à une concentration entre 5 et 200 mM et un pH entre 2,8 et 6; et
facultativement où le vecteur pharmaceutiquement acceptable comprend une concentration totale d'acétate de sodium et d'acétate de calcium entre 50 mM et 500 mM.

27. Composition antifolate liposomale selon la revendication 21, où l'agent antifolate bioactif est soluble dans l'eau.

28. Composition antifolate liposomale selon la revendication 21, où chaque liposome comprend moins de 200 000 molécules de l'agent antifolate bioactif; ou entre 10 000 et 100 000 de l'agent antifolate bioactif.

29. Composition antifolate liposomale selon la revendication 21, où l'agent antifolate bioactif est
le pémétrexed;
le lométrexol;
au moins un choisi dans le groupe consistant en méthotrexate; ralitrexed; aminoptérine; pralatrexate; lométrexol; analogue thiophène du lométrexol; analogue furane du lométrexol; trimétrexed; LY309887; et GW 1843U89; ou
au moins un choisi dans le groupe consistant en proguanil; pyriméthamine; triméthoprime; et Pyrrolo 6-Substitué et un membre de la classe des Thieon[2,3-d]pyrrolopyrimidines d'inhibiteurs de GARFT.

30. Composition antifolate liposomale selon la revendication 21, où l'agent antifolate bioactif est à un pH de 5 à 8 ou à un pH de 2 à 6.

31. Composition antifolate liposomale selon la revendication 21, où la protéine est liée de manière covalente via un groupe fonctionnel maléimide à une molécule de stabilisant stérique.

32. Composition antifolate liposomale selon la revendication 21, où la protéine a une affinité spécifique pour au moins un, au moins deux ou tous les trois choisis dans le groupe consistant en: récepteur de folate alpha; récepteur de folate bêta; et récepteur de folate delta.

33. Composition antifolate liposomale selon la revendication 21, où la protéine a une affinité spécifique pour un épitope sur un antigène de surface de cellule tumorale qui est présent sur une cellule tumorale mais absent ou inaccessible sur une cellule non tumorale;
facultativement où ladite cellule tumorale est une cellule maligne; ou
facultativement où l'antigène de surface de cellule tumorale est au moins un choisi dans le groupe consistant en: récepteur de folate alpha; récepteur de folate bêta; et récepteur de folate delta.

34. Composition antifolate liposomale selon la revendication 21, où la protéine comprend une séquence liant un antigène d'un anticorps;
facultativement où la séquence liant un antigène d'un anticorps comprend une ou plusieurs régions déterminant la complémentarité d'origine anticorps; ou
facultativement où la protéine est au moins une choisie dans le groupe consistant en un anticorps; un anticorps humanisé; un fragment liant un antigène d'un anticorps; un anticorps à chaîne unique; un anticorps à domaine unique; un anticorps bispécifique; un anticorps synthétique; un anticorps pégylé; et un anticorps multimère.

35. Composition antifolate liposomale selon la revendication 21, où chaque liposome comprend jusqu'à 200 entités de ciblage; facultativement chaque liposome comprend de 30 à 200 entités de ciblage.

36. Composition antifolate liposomale selon la revendication 21 destinée à être utilisée dans le traitement du cancer.

37. Procédé de préparation d'une composition selon la revendication 25 comprenant:
la formation d'un mélange comprenant:
des composants liposomaux comprenant au moins un choisi dans le groupe consistant en: DSPE; DSPE-PEG; DSPE-maléimide; HSPC; HSPC-PEG; HSPC-maléimide; cholestérol; cholestérol-PEG; et cholestérol-maléimide; et
au moins un choisi dans le groupe consistant en: DSPE; DSPE-FITC; DSPE-maléimide; cholestérol; et HSPC;
l'agent antifolate bioactif en solution aqueuse;
la protéine;
l'homogénéisation du mélange pour former des liposomes dans ladite solution aqueuse; et
l'extrusion du mélange à travers une membrane pour former des liposomes renfermant l'agent antifolate bioactif dans une solution aqueuse;
facultativement le retrait de l'agent antifolate bioactif en excès en solution aqueuse à l'extérieur des liposomes après ladite étape d'extrusion, en outre facultativement la lyophilisation de ladite composition après ladite étape de retrait pour former une composition lyophilisée, et en outre facultativement la reconstitution de ladite composition de lyophilisation par dissolution de ladite composition de lyophilisation dans un solvant après ladite étape de lyophilisation, et en outre facultativement où ledit solvant est un solvant aqueux; et
facultativement où le mélange comprend au moins un choisi dans le groupe consistant en mannitol; tréhalose; sorbitol; et saccharose.

38. Procédé selon la revendication 37, où les liposomes formés comprennent en outre un stabilisant stérique, où le stabilisant stérique est au moins un choisi dans le groupe consistant en polyéthylèneglycol (PEG); poly-L-lysine (PLL); monosialoganglioside (GM1); poly(vinylpyrrolidone) (PVP); poly(acrylamide) (PAA); poly(2-méthyl-2-oxazoline); poly(2-éthyl-2-oxazoline); phosphatidyl polyglycérol; poly[*N*-(2-hydroxypropyl)-méthacrylamide]; les poly-*N*-vinylpyrrolidones amphiphiles; un polymère à base de L-aminoacides; et poly(alcool vinylique); et facultativement où le au moins un stabilisant est le PEG et ledit PEG a un poids moléculaire moyen en nombre (Mn) de 200 à 5000 daltons.

39. Composition liposomale ciblée qui cible sélectivement les récepteurs de folate comprenant:
un liposome incluant un espace intérieur;
un agent bioactif disposé dans ledit espace intérieur;
une molécule de stabilisant stérique fixée à un extérieur du liposome; et
une protéine ayant une affinité spécifique pour au moins un récepteur de folate, ladite protéine fixée à au moins l'un du stabilisant stérique et de l'extérieur du liposome;
où le liposome a un potentiel zêta qui est inférieur ou égal à zéro et le liposome est anionique ou neutre;
facultativement où le stabilisant stérique est au moins un choisi dans le groupe consistant en polyéthylèneglycol (PEG); poly-L-lysine (PLL); monosialoganglioside (GM1); poly(vinylpyrrolidone) (PVP); poly(acrylamide) (PAA); poly(2-méthyl-2-oxazoline); poly(2-éthyl-2-oxazoline); phosphatidyl polyglycérol; poly[*N*-(2-hydroxypropyl)méthacrylamide]; les poly-*N*-vinylpyrrolidones amphiphiles; un polymère à base de L-aminoacides; et poly(alcool vinylique), facultativement où lorsque le stabilisant stérique est le PEG et le PEG a un poids moléculaire moyen en nombre (Mn) de 200 à 5000 daltons; et
facultativement où l'agent bioactif comprend au moins l'un du groupe consistant en ellipticine; paclitaxel; pémétrexed; méthotrexate; ralitrexed; aminoptérine; pralatrexate; lométrexol; trimétrexed; LY309887; GW 1843U89; proguanil; pyriméthamine; triméthoprime et Pyrrolo 6-substitué et la classe des Thieon[2,3-d]pyrrolopyrimidines d'inhibiteurs de GARFT.

40. Composition antifolate liposomale selon la revendication 39 destinée à être utilisée dans le traitement du cancer.

41. Procédé de préparation d'une composition selon la revendication 39 où ledit liposome est formé, comprenant les étapes de:
formation d'un mélange comprenant:
des composants liposomaux comprenant au moins un choisi dans le groupe consistant en: DSPE; DSPE-PEG, DSPE-maléimide; HSPC; HSPC-PEG; HSPC-maléimide; cholestérol; cholestérol-PEG; et cholestérol-maléimide; et au moins un choisi dans le groupe consistant en: DSPE; DSPE-FITC; DSPE-maléimide; cholestérol; et HSPC;
l'agent bioactif en solution aqueuse;
la protéine;
homogénéisation du mélange pour former des liposomes dans ladite solution aqueuse; et
extrusion du mélange à travers une membrane pour former des liposomes renfermant l'agent antifolate bioactif dans une solution aqueuse;
facultativement retrait de l'agent antifolate bioactif en excès en solution aqueuse à l'extérieur des liposomes après ladite étape d'extrusion, en outre facultativement lyophilisation de ladite composition après ladite étape de retrait pour former une composition lyophilisée, et en outre facultativement reconstitution de ladite composition de lyophilisation par dissolution de ladite composition de lyophilisation dans un solvant après ladite étape de lyophilisation, et en outre facultativement où ledit solvant est un solvant aqueux; et
facultativement où le mélange comprend au moins un choisi dans le groupe consistant en mannitol; tréhalose; sorbitol; et saccharose.

42. Procédé selon la revendication 41, où les liposomes formés comprennent en outre un stabilisant stérique; où le stabilisant stérique est au moins un choisi dans le groupe consistant en polyéthylèneglycol (PEG); poly-L-lysine (PLL); monosialoganglioside (GM1); poly(vinylpyrrolidone) (PVP); poly(acrylamide) (PAA); poly(2-méthyl-2-oxazoline); poly(2-éthyl-2-oxazoline); phosphatidyl polyglycérol; poly[*N*-(2-hydroxypropyl)-méthacrylamide]; les poly-*N*-vinylpyrrolidones amphiphiles; un polymère à base de L-aminoacides; et poly(alcool vinylique); et facultativement où le au moins un stabilisant est le PEG et le PEG a un poids moléculaire moyen en nombre (Mn) de 200 à 5000 daltons.

43. Kit pour fournir une composition selon la revendication 25 comprenant:
des composants liposomaux comprenant au moins un choisi dans le groupe consistant en: DSPE; DSPE-PEG, DSPE-maléimide; HSPC; HSPC-PEG; HSPC-maléimide; cholestérol; cholestérol-PEG; et cholestérol-maléimide; et au moins un choisi dans le groupe consistant en: DSPE; DSPE-FITC; DSPE-maléimide; cholestérol; et HSPC,
une instruction pour utiliser la composition pour encapsuler un agent bioactif, et facultativement, dans un récipient séparé, l'agent bioactif.
